(19) Europäisches Patentamt
European Patent Office
Office européen des brevets

(11) **EP 1 782 772 A1**

(12) **EUROPEAN PATENT APPLICATION**
published in accordance with Art. 158(3) EPC

(43) Date of publication:
**09.05.2007 Bulletin 2007/19**

(51) Int Cl.:
***A61F 7/08*** *(2006.01)*    ***C09K 5/16*** *(2006.01)*

(21) Application number: **05765765.2**

(22) Date of filing: **14.07.2005**

(86) International application number:
**PCT/JP2005/013001**

(87) International publication number:
**WO 2006/006648 (19.01.2006 Gazette 2006/03)**

(84) Designated Contracting States:
**DE FR GB**

(30) Priority: **14.07.2004 JP 2004207829**

(71) Applicant: **Mycoal Products Corporation**
**Tochigi-chi, Tochigi 328-0067 (JP)**

(72) Inventor: **Dodo, Toshihiro,**
**c/o Mycoal Products Corp.**
**Tochigi-shi, Tochigi 3280067 (JP)**

(74) Representative: **Thun, Clemens et al**
**Mitscherlich & Partner**
**Sonnenstrasse 33**
**80331 München (DE)**

(54) **EXOTHERMAL COMPOSITION, EXOTHERMAL ARTICLE AND METHOD FOR PRODUCING EXOTHERMAL ARTICLE**

(57)    There are provided a heat generating composition and a heat generating body having excellent exothermic rising properties and having exothermic holding properties over a long period of time and a process for producing the same.

The invention is concerned with a heat generating composition, **characterized in that** the heat generating composition contains, as essential components, an iron powder, a carbon component, a reaction accelerator, a hydrogen formation inhibitor and water; that the iron powder contains from 20 to 100 % by weight of an active iron powder; and that the active iron powder is at least any one of an active iron powder in which at least a part of the surface of an iron powder is covered by an iron oxide film and a thickness of the iron oxide film is 3 nm or more, and which has a region of an oxygen-free iron component in at least one region selected from a central region of the active iron powder and a region beneath the iron oxide film, and an active iron powder in which an amount of wustite is from 2 to 50 % by weight in terms of an X-ray peak intensity ratio to iron.

*FIG.2*

**Description**

[Technical Field]

The present invention relates to a heat generating composition using an active iron powder in which at least a part of the surface of an iron powder is covered by an iron oxide film and a thickness of the iron oxide film is 3 nm or more and to a heat generating body using the same. In more detail, the invention relates to a heat generating composition capable of causing an exothermic reaction immediately upon contact with air, having a fast temperature rise rate by the heat generation and having excellent exothermic rising properties and to a heat generating body using the same.

[Background Art]

In general, throwaway body warmers are well known as a product to be used by the action of a mixture of an iron powder and a reaction aid, etc. with air (oxygen).

As a metal powder to be used in such a product, it is known that an iron powder is the most general. Salt, water, or the like is used as the reaction aid, and it is also well known that active carbon, vermiculite, diatomaceous earth, wood meal, or a water absorptive polymer is mixed as a water retaining agent for carrying such a substance thereon and used.

In the throwaway body warmer, it is desired that the temperature increases promptly after opening the seal (for example, see Patent Documents 1 and 2).

Patent Document 1 proposes a heat generating composition in which manganese dioxide, cupric oxide and triiron tetroxide are mixed with other heat generating composition components. These substances function as a catalyst and cannot be used as an exothermic substance. Furthermore, in the case where these substances are added in a heat generating composition, the contact between triiron tetroxide, etc. and the surface of an iron powder is not sufficient so that the described effects are not so revealed. In general, in a low temperature region of from about 30 °C to about 90 °C in which a heat generating body is used for the purpose of warming a human body or a body, these substances are not substantially effective in exothermic rising properties, and when the proportion of addition of the substances increases, there was encountered a problem such that the exothermic duration time becomes short.

Patent Document 2 proposes a heat generating body in which after reaching 40 °C, a heat generating composition containing, as major components, iron, water and an oxidation promoter such as salt is accommodated in an air-permeable container. However, it takes 25 minutes for making this heat generating composition reach 40 °C, and therefore, industrial mass production thereof was difficult.

Furthermore, in order to obtain a more comfortable feeling for use, there are proposed a variety of heat generating compositions designing to keep moldability by using a thickener, a binding agent, etc. for the purposes of preventing deviation of a heat generating composition and designing fitness by various shapes. For example, Patent Document 3 proposes a throwaway body warmer comprising a heat generating composition having shape holding properties by adding a powdered thickener such as corn starch and potato starch.

Furthermore, Patent Document 4 proposes a solid heat generating composition prepared by mixing a binding agent such as CMC in a powdered heat generating composition and compression molding the mixture.

Furthermore, Patent Document 5 proposes an ink-like and/or creamy heat generating composition to which viscosity is given by using a thickener and a heat generating body and a process for producing the same.

With respect to such ink-like and/or creamy viscous heat generating compositions or adhesive and flocculating heat generating compositions, in those compositions using a thickener such as glue, gum arabic, and CMC, a flocculant aid such as α-starch, an excipient, and a binding agent, particles of the heat generating composition are coupled by such viscosity imparting substances. Thus, though these compositions were excellent with respect to prevention of deviation and moldability, their exothermic performance was remarkably deteriorated. Similarly, in viscous heat generating compositions as prepared by using a thickener or a binding agent, particles of the heat generating composition are coupled by using the thickener or binding agent. Thus, though these compositions were excellent with respect to prevention of deviation and moldability, their exothermic performance was remarkably deteriorated.

That is, even when liberated water is absorbed in a support, a covering material, a water absorptive material, etc., the heat generating composition is viscous due to the binding agent, the thickener, the flocculant aid, the excipient, or the like. Thus, the reaction became slow, or a rapid temperature rise to a prescribed temperature or warming over a long period of time was difficult because the liberated water hardly comes out, or the thickener or the like adversely affects the exothermic substances.

Furthermore, conventionally used powdered or particulate heat generating compositions do not have moldability because surplus water is not present or insufficient. Although these powdered or particulate heat generating compositions are good in exothermic characteristics, since a heat generating body is formed by filling such a composition in an air-permeable accommodating bag, the exothermic temperature distribution is not constant due to deviation of the heat generating composition or the like, the feeling for use is worse, and it is difficult to produce a heat generating body having a shape adaptive to the shape of a body to be heat insulated. Thus, their exothermic performance could not be sufficiently exhibited.

[Patent Document 1] JP-A-53-60885
[Patent Document 2] JP-A-57-10673
[Patent Document 3] JP-A-6-343658
[Patent Document 4] JP-A-59-189183
[Patent Document 5] JP-A-9-75388

[Disclosure of the Invention]

[Problems that the Invention is to Solve]

The invention is to provide a heat generating composition and a heat generating body each having excellent exothermic rising properties and having exothermic holding properties over a long period of time and a process for producing the same.

[Means for Solving the Problems]

Specifically, as set forth in claim 1, a heat generating composition of the invention is characterized in that the heat generating composition contains, as essential components, an iron powder, a carbon component, a reaction accelerator, a hydrogen formation inhibitor and water; that the iron powder contains from 20 to 100 % by weight of an active iron powder; and that the active iron powder is at least any one of:

1) an active iron powder comprising particles, a surface of each of which is at least partially covered with an iron oxide film and a thickness of the iron oxide film is 3 nm or more, and which has a region of an oxygen-free iron component in at least one region selected from a central region of the particle and a region beneath the iron oxide film, and

2) an active iron powder in which an amount of wustite is from 2 to 50 % by weight in terms of an X-ray peak intensity ratio to iron.
Also, a heat generating composition as set forth in claim 2 is characterized in that in the heat generating composition as set forth in claim 1, the amount of wustite of the active iron powder is from 5.01 to 50 % by weight in terms of an X-ray peak intensity ratio to iron.
Also, a heat generating composition as set forth in claim 3 is characterized in that in the heat generating composition as set forth in claim 1, the heat generating composition contains at least one member selected from additional components consisting of a water retaining agent, a water absorptive polymer, a pH adjusting agent, an aggregate, a fibrous material, a functional substance, a surfactant, an organosilicon compound, a pyroelectric substance, a moisturizer, a fertilizer component, a hydrophobic polymer compound, a heat generating aid, a metal other than iron, a metal oxide other than iron oxide, an acidic substance, and a mixture thereof.
Also, a heat generating composition as set forth in claim 4 is characterized in that in the heat generating composition as set forth in claim 1, the heat generating composition has a water mobility value showing an amount of surplus water of less than 0.01.
Also, a heat generating composition as set forth in claim 5 is characterized in that in the heat generating composition as set forth in claim 1, the heat generating composition contains surplus water having a water mobility value of from 0.01 to 20 and has moldability by the surplus water; and that the surplus water in the heat generating composition does not reveal a function as a barrier layer, whereby an exothermic reaction with air is not inhibited.
Also, a heat generating composition as set forth in claim 6 is characterized in that in the heat generating composition as set forth in claim 1, the heat generating composition contains surplus water having a water mobility value of exceeding 20 and not more than 50 and has moldability by the surplus water; and that the surplus water in the heat generating composition reveals a function as a barrier layer, whereby an exothermic reaction with air is inhibited.
As set forth in claim 7, a heat generating body of the invention is characterized in that the heat generating composition as set forth in claim 1 is accommodated in an air-permeable accommodating bag to form an exothermic part.
Also, a heat generating body as set forth in claim 8 is characterized in that in the heat generating body as set forth in claim 7, the accommodated heat generating composition is a heat generating composition molded body.
Also, a heat generating body as set forth in claim 9 is characterized in that in the heat generating body as set forth in claim 7, the accommodated heat generating composition forms sectional exothermic parts sectioned into plural sections by a seal part within the air-permeable accommodating bag; and that an exothermic part is formed from gathering of the sectional exothermic parts.

Also, a heat generating body as set forth in claim 10 is characterized in that in the heat generating body as set forth in claim 9, the sectional exothermic parts have a longest width of from 1 to 55 mm and a maximum height of from 0.1 to 10 mm; that a space of the sectional exothermic parts is from 0.5 to 30 mm; that the surroundings of the heat generating composition molded body are heat sealed to form sectional exothermic parts; that at least a part of the accommodating bag has air permeability; and that the periphery which becomes the heat generating body is sealed.

Also, a heat generating body as set forth in claim 11 is characterized in that in the heat generating body as set forth in claim 9, the heat generating composition molded body has a curved shape; and that the sectional exothermic parts have a curved shape and have a shortest radius of curvature of from 0.5 to 27.5 mm and a height of from 0.1 to 15 mm.

Also, a heat generating body as set forth in claim 12 is characterized in that in the heat generating body as set forth in claim 7, a fixing measure is provided on the exposed surface of the heat generating body and optionally provided with a separator.

As set forth in claim 13, a process for producing a heat generating body of the invention is characterized by accommodating a heat generating composition which contains, as essential components, an iron powder containing from 20 to 100 % by weight of at least one of:

1) an active iron powder comprising particles, a surface of each of which is at least partially covered with an iron oxide film and a thickness of the iron oxide film is 3 nm or more, and which has a region of an oxygen-free iron component in at least one region selected from a central region of the particles and a region beneath the iron oxide film, and
2) an active iron powder in which an amount of wustite is from 2 to 50 % by weight in terms of an X-ray peak intensity ratio to iron,
a carbon component, a reaction accelerator, a hydrogen formation inhibitor and water in an air-permeable accommodating bag.

Also, a process for producing a heat generating body as set forth in claim 14 is characterized in that in the process for producing a heat generating body as set forth in claim 13, the heat generating composition contains at least one member selected from additional components consisting of a water retaining agent, a water absorptive polymer, a pH adjusting agent, a surfactant, an organosilicon compound, a hydrophobic polymer compound, a pyroelectric substance, an antioxidant, an aggregate, a fibrous material, a moisturizer, a functional substance, and a mixture thereof.

Also, a process for producing a heat generating body as set forth in claim 15 is characterized in that in the process for producing a heat generating body as set forth in claim 13, the heat generating composition as accommodated in the air-permeable accommodating bag forms sectional exothermic parts sectioned into plural sections by a seal part within the air-permeable accommodating bag.

Also, a process for producing a heat generating body as set forth in claim 16 is characterized in that in the process for producing a heat generating body as set forth in claim 13, the heat generating composition is a molded body, is provided in a packaging material made of a substrate and a covering material constituting the air-permeable accommodating bag and seals the surroundings of the heat generating composition molded body to produce a heat generating body.

Also, in the heat generating body, it is preferable that the heat generating composition molded body is compressed.

Also, in the heat generating body, it is preferable that the sectioned part of the sectional exothermic parts is provided with a perforation.

Also, in the heat generating body, it is preferable that the fixing measure is an adhesive layer; and that the adhesive layer contains at least one member selected from additional components consisting of a water retaining agent, a water absorptive polymer, a pH adjusting agent, a surfactant, an organosilicon compound, a hydrophobic polymer compound, a pyroelectric substance, an antioxidant, an aggregate, a carbon component, a fibrous material, a moisturizer, a functional substance, and a mixture thereof.

Also, in the heat generating body, it is preferable that the adhesive layer is a non-hydrophilic adhesive layer; and that an adhesive constituting the layer is a non-aromatic hot melt based adhesive.

Also, in the heat generating body, it is preferable that the adhesive layer is a hydrophilic adhesive layer.

[Advantages of the Invention]

In the heat generating composition of the invention, since a heat generating composition containing an active iron powder is used, the following advantages are obtainable.

1) The heat generating composition of the invention is excellent in exothermic rising properties and is able to be warmed faster 2 times or more as compared with conventional products, whereby a heat generating body which has caused thorough heat generation is immediately obtained.

2) Since the heat generating composition of the invention has satisfactory exothermic rising properties, the amount of use of a carbon component such as active carbon can be reduced by 10 to 20 % or more as compared with conventional products which do not use an active iron powder.

3) Since the heat generating composition of the invention is moldable, various molding methods such as a filling system and a force-through system can be employed. Furthermore, since both filling and lamination are possible and a production rate over the range of from a low rate to a high rate can be employed, it is possible to employ a production system in response to the production circumstances.

4) Since the heat generating composition of the invention is moldable, it is possible to employ various types over the range of from an ultra-thin type to a thick type, various shapes over the range of from lines to curves such as a rectangular shape, a foot shape, a dumbbell shape, a point shape and a linear shape, and various sizes over the range of from an extra fine size to a large size.

5) Since the heat generating composition of the invention is excellent in compression processability such that a lowering in exothermic characteristics caused by compression of the heat generating composition can be remarkably prevented, it is possible to hold exothermic characteristics even when formed as an ultra-thin type heat generating body over a long period of time.

6) In a material obtained by appropriately compressing a molded body of the heat generating composition under pressure, even when a perforated film which is difficult with respect to the pressure adjustment is used as a raw material of an air-permeable part in place of a porous film, or even when an inner pressure of the accommodating bag becomes equal to or more than an outer pressure, shape collapse hardly occurs so that it is possible to use a perforated film. Accordingly, not only the range for selecting an air-permeable raw material is widened so that the costs can be lowered, but also a body to be warmed can be uniformly warmed at an appropriate temperature over a long period of time.

7) Heat generating compositions over the range of from a heat generating composition containing surplus water to a heat generating composition not containing surplus water can be easily produced by adjusting production conditions during the production step. Accordingly, it becomes possible to produce heat generating bodies over a wide range such as a heat generating body which is not heat generated during the production but is heat generated after the production and a heat generating body using a non-water absorptive accommodating bag.

[Best Modes for Carrying Out the Invention]

The heat generating composition of the invention is a heat generating composition which is characterized in that the heat generating composition contains, as essential components, an iron powder, a carbon component, a reaction accelerator, a hydrogen formation inhibitor and water; that the iron powder contains from 20 to 100 % by weight of an active iron powder; and that the active iron powder is at least any one of:

1) an active iron powder in which at least a part of the surface of an iron powder is covered by an iron oxide film and a thickness of the iron oxide film is 3 nm or more, and which has a region of an oxygen-free iron component in at least one region selected from a central region of the active iron powder and a region beneath the iron oxide film, and

2) an active iron powder in which an amount of wustite is from 2 to 50 % by weight in terms of an X-ray peak intensity ratio to iron.

In this way, the invention is able to put a heat generating composition and a heat generating body each having excellent exothermic rising properties and having exothermic holding properties over a long period of time into practical use.

The carbon component is not limited so far as it is a component made of carbon. Examples thereof include conductive graphite, carbon black, graphite, active carbon, carbon nanotubes, carbon nanohorns, and fullerenes. Examples also include active carbons prepared from a coconut shell, a wood meal, charcoal, coal, and bone coal; and ones prepared from other raw materials such as animal products, natural gases, fats, oils, and resins. Of these, active carbons having an adsorption holding ability are especially preferable. The fullerenes include ones which have become conductive by doping.

Furthermore, the carbon component is not always required to be present singly. In the case where an iron powder which contains a carbon component and/or is covered by a carbon component is used in a heat generating composition, it is to be noted that even when the carbon component is not present singly, the heat generating composition contains a carbon component.

The reaction accelerator is not particularly limited so far as it is able to promote the reaction of the heat generating substance. Examples thereof include metal halides, nitrates, acetates, carbonates, and metal sulfates. Examples of metal halides include sodium chloride, potassium chloride, magnetic chloride, calcium chloride, ferrous chloride, ferric chloride, sodium bromide, potassium bromide, ferrous bromide, ferric bromide, sodium iodide, and potassium iodide. Examples of nitrates include sodium nitrate and potassium nitrate. Examples of acetates include sodium acetate. Examples of carbonates include ferrous carbonate. Examples of metal sulfates include potassium sulfate, sodium sulfate, and ferrous sulfate.

The hydrogen formation inhibitor is not limited so far as it inhibits the formation of hydrogen. Examples thereof include at least one or two or more members selected from the group consisting of sulfur compounds, oxidizing agents, alkaline substances, sulfur, antimony, selenium, phosphorus, and tellurium.

The sulfur compound is a compound with an alkali metal or alkaline earth metal, and examples thereof include metal sulfides such as calcium sulfide, metal sulfites such as sodium sulfite, and metal thiosulfates such as sodium thiosulfate. Examples of the oxidizing agent include nitrates, oxides, peroxides, halogenated hydroacid salts, permanganates, and chromates.

The alkaline substance is not limited so far as it is a substance exhibiting alkaline properties. Examples thereof include silicates, phosphates, sulfites, thiosulfates, carbonates, hydrogencarbonates, hydroxides, $Na_3PO_4$, and $Ca(OH)_2$.

By adding an appropriate amount of this hydrogen formation inhibitor, nevertheless a heat generating composition having high activity, the formation of a gas is inhibited; even when a heat generating body is accommodated in an outer bag which is an air-impermeable accommodating bag and then stored, transported or placed in the shop, swelling of the outer bag does not occur. Thus, it has become possible to provide a heat generating body with high performance.

As the hydrogen formation inhibitor, combinations of a sulfur compound and a hydroxide of an alkali metal or alkaline earth metal are especially preferable.

As the water, one from a proper source may be employed. Its purity and kind and the like are not particularly limited.

In the case of the heat generating composition, the content of water is preferably from 1 to 60 % by weight, more preferably from 1 to 40 % by weight, further preferably from 7 to 40 % by weight, still further preferably from 10 to 35 % by weight, and even further preferably from 20 to 30 % by weight of the heat generating composition.

Furthermore, in the case of the reaction mixture or heat generating mixture prior to the contact treatment with an oxidizing gas, the content of water is preferably from 0.5 to 20 % by weight, more preferably from 1 to 20 % by weight, further preferably from 5 to 20 % by weight, and still further preferably from 7 to 15 % by weight of the reaction mixture or heat generating mixture.

The iron powder is an iron powder containing from 20 to 100 % by weight of an active iron powder.

Examples of an iron powder capable of forming an active iron powder and/or an iron powder which is not an active iron powder include a cast iron powder, an atomized iron powder, an electrolyzed iron powder, a reduced iron powder, and iron alloys thereof.

The iron powder or active iron powder may contain a metal other than iron, a semiconductor, or an oxide thereof.

The "iron alloy powder" as referred to herein is an iron alloy powder containing 50 % or more of iron. The alloy component is not particularly limited so far as it is a metal component including semiconductors other than iron and the iron component functions as a component of the heat generating composition, and examples thereof include silicon, zinc, aluminum, magnesium, manganese, nickel, and copper.

The iron powder may be an iron powder which contains a carbon component and/or is covered by a carbon component. An iron powder or an iron alloy powder in which an iron component thereof is an iron powder or an iron alloy powder, the surface of which is partially covered by from 0.3 to 3.0 % by weight of a conductive carbonaceous substance, is especially useful.

As the metal oxide other than iron oxide in the iron component which contains oxygen and/or is covered by oxygen, any substance may be employed so far as it does not hinder the oxidation of iron by an oxidizing gas. Examples thereof include manganese dioxide and cupric oxide.

The "active iron powder" as referred to herein is an active iron powder in which at least a part of the surface of an iron powder is covered by an iron oxide film and one of which has a thickness of the iron oxide film of 3 nm or more and has a region of an oxygen-free iron component in at least one region selected from a central region of an active iron powder and a region beneath of the iron oxide film.

A thickness of the iron oxide film which is an oxygen-containing film which covers the surface of the iron powder is usually from 3 nm or more, preferably from 3 nm to 100 $\mu$m, more preferably from 30 to 100 $\mu$m, further preferably from 30 nm to 50 $\mu$m, still further preferably from 30 nm to 1 $\mu$m, even further preferably from 30 nm to 500 nm, and even still further preferably from 50 nm to 300 nm by using the Auger electron spectroscopy. When the thickness of the oxygen-containing film of iron is 3 nm or more, the oxygen-containing film of iron is able to exhibit an effect for promoting an oxygen reaction, and upon contact with an oxidizing gas such as air, it is possible to immediately initiate the oxidation reaction.

When the thickness of the oxygen-containing film of iron is 100 nm or more, though there is some possibility that the exothermic time is shortened, such can be employed depending upon the utility.

Furthermore, another is an active iron powder having wustite, and an amount of wustite is usually from 2 to 50 % by weight, preferably from 5.01 to 50 % by weight, more preferably from 5.01 to 40 % by weight, further preferably from 6 to 40 % by weight, still further preferably from 7 to 30 % by weight, and even further preferably from 7 to 25 % by weight in terms of an X-ray peak intensity ratio to iron. Even when the amount of wustite exceeds 50 % by weight, though the exothermic rising properties are satisfactory, an exothermic duration becomes short. When the amount of wustite is less than 2 % by weight, the exothermic rising properties become dull.

Furthermore, needless to say, in the active iron powder of the invention, an iron oxide film which is a film made of oxygen-containing iron such as oxides, hydroxides and oxyhydroxides of iron is present on the surface of an iron powder. However, in a heat generating composition having other components added thereto, the surface thereof is at least partially oxidized, and a reaction active part composed mainly of an oxide, a hydroxide, a chlorine ion, a hydrogen ion, etc. is generated, whereby exothermic reactivity and hydrophilicity are improved, and moldability, exothermic rising properties and a utilization factor of iron are remarkably improved. It is thought that since the surface of hydrated iron oxide (amorphous) has chemical activity and adsorbs other substances such as water, the partially oxidized iron powder generates hydrophilicity, the moldability is improved, and the iron oxide compound which constitutes the iron oxide film exhibits an effect for improving the exothermic rising properties.

A process for producing the active iron powder or iron powder containing from 20 to 100 % by weight of an active iron powder is not limited. Examples thereof include a contact treatment with an oxidizing gas in which a reaction mixture having components of a heat generating composition mixed therein or a heat generating composition is brought into continuous or intermittent contact with an oxidizing gas (for example, oxygen and air) in an oxidizing gas atmosphere or by blowing an oxidizing gas, or the like, thereby partially oxidizing the iron component. A method for determining a degree of oxidation is not limited. Examples thereof include a method of determining a degree of contact of the reaction mixture or heat generating composition with an oxidizing gas by a water mobility value of the reaction mixture or heat generating composition, a contact time with an oxidizing gas, an exothermic temperature rise rate at the time of contact, an exothermic temperature at the time of contact, a maximum exothermic temperature at the time of contact, a prescribed temperature as dropped after reaching a maximum exothermic temperature at the time of contact, or a combination thereof, thereby determining a degree of oxidation.

In addition, the following can be specifically enumerated.

(1) A production process by reducing a mill scale or an ore to be used as a raw material of an iron powder at a temperature of not higher than about 1,300 °C by using a reducing agent such as hydrogen, charcoal, and coke, coarsely pulverizing the reduced cake by a hammer mill, a jaw crusher, etc., and then finely pulverizing it by a novorotor, a pulverizer, or a vibration bowl.

(2) A production process by reducing an iron powder containing an iron oxide, thereby producing a partially oxidized iron powder.

(3) A production process by exposing and allowing an iron powder to stand in air, thereby producing a partially oxidized iron powder.

(4) A production process by exposing and allowing a mixture of an iron powder, a reaction accelerator and water to stand in air, thereby producing a partially oxidized iron powder.

(5) A production process by subjecting a reaction mixture of an iron powder, a reaction accelerator and water in an oxidizing gas atmosphere to a self-exothermic reaction to partially oxidize the iron powder, thereby producing a partially oxidized iron powder.

(6) A production process by subjecting a reaction mixture of an iron powder, a reaction accelerator, an acidic substance and water in an oxidizing gas atmosphere to a self-exothermic reaction to partially oxidize the iron powder, thereby producing a partially oxidized iron powder.

(7) A production process by subjecting a reaction mixture of an iron powder, a reaction accelerator, a carbon component and water in an oxidizing gas atmosphere to a self-exothermic reaction to partially oxidize the iron powder, thereby producing a partially oxidized iron powder.

(8) A production process by subjecting a reaction mixture of an iron powder, a reaction accelerator, an acidic substance, a carbon component and water in an oxidizing gas atmosphere to a self-exothermic reaction to partially oxidize the iron powder, thereby producing a partially oxidized iron powder.

(9) A production process by bringing a reaction mixture containing, as essential components, an iron powder, a reaction accelerator, a carbon component and water and having a water content of from 1 to 30 % by weight and a water mobility value of less than 0.01 into contact with an oxidizing gas and holding the temperature of the reaction mixture at the time of contact at 40 °C or higher for 2 seconds or more, thereby producing an active iron powder.

(10) A production process by containing other component than the essential components in the reaction mixture as set forth above in any one of (7) to (9), thereby producing a partially oxidized iron powder.

Incidentally, the term "other component than the essential components" as referred to herein means at least one member selected from additional components consisting of a water retaining agent, a water absorptive polymer, a pH adjusting agent, a hydrogen formation inhibitor, an aggregate, a fibrous material, a functional substance, a surfactant, an organosilicon compound, a pyroelectric substance, a moisturizer, a fertilizer component, a hydrophobic polymer compound, a heat generating aid, a metal other than iron, a metal oxide other than iron oxide, an acidic substance, and a mixture thereof. If desired, a magnetic body may be further added.

(11) A production process by carrying out a process as set forth above in any one of (1) to (5) by using a reaction mixture having a water mobility value of less than 0.01, thereby producing a partially oxidized iron powder.

(12) A production process by carrying out a process as set forth above in any one of (1) to (6) by using a reaction mixture having a water mobility value of less than 0.01 and a water content of from 0.01 to 20 % by weight, thereby producing a partially oxidized iron powder.

(13) A production process by carrying out a process as set forth above in any one of (1) to (5) by using a reaction mixture having a water mobility value of 0.01 or more, thereby producing a partially oxidized iron powder.

(14) A production process by carrying out a process as set forth above in any one of (1) to (8) by warming at 10 °C or higher, thereby producing a partially oxidized iron powder.

(15) A production process by carrying out a process as set forth above in any one of (1) to (8) by blowing an oxidizing gas, thereby producing a partially oxidized iron powder.

(16) A production process by carrying out a process as set forth above in (11) by blowing an oxidizing gas warmed at 10 °C or higher, thereby producing a partially oxidized iron powder.

(17) A production process by carrying out a process as set forth above in any one of (1) to (12), wherein the water content in the mixture prior to the contact treatment with an oxidizing gas is from 0.5 to 30 %, and the contact treatment with an oxidizing gas is carried out until the temperature reaches a maximum temperature which is a maximum point of a temperature rise due to the exothermic reaction or exceeds the maximum temperature, thereby producing a partially oxidized iron powder (in this case, it is preferable that the contact treatment with an oxidizing gas is carried out until the temperature drops by at least 10 to 20 °C from the maximum temperature).

Incidentally, the circumstances of the reaction mixture part at the time of contact treatment with an oxidizing gas are not limited. Examples thereof include a state that the reaction mixture part is present in a lid-free vessel of an open system and a state that an oxidizing gas such as air comes into a vessel through an air-permeable sheet-like material such as non-woven fabrics. Furthermore, the contact treatment with an oxidizing gas may be carried out with stirring or without stirring and may be carried out in a batchwise system or continuous system.

As a method for analyzing the thickness of the iron oxide film of the active iron powder, the Auger electron spectroscopy is employed, and for measuring the amount of wustite, the X-ray diffraction method is employed.

The "thickness of the iron oxide film" as referred to herein means a portion in which in the case of sputtering the surface of the iron powder with Ar at a sputtering rate of 11 nm/min as reduced into Fe in the depth direction by using the Auger electron spectroscopy, a ratio ($Io/Ii$) of a peak intensity of O ($Io$) to a peak intensity of Fe ($Ii$) is 0.05 or more. Accordingly, the thickness of the oxygen-containing film of iron on the surface of the iron powder is a distance, as reduced into Fe, from the surface of the iron powder to a depth at which ($Io/Ii$) is 0.05. With respect to the measurement condition, the sputtering time is 15 minutes, and the sputtering rate is 11 nm/min (as reduced into Fe). With a lapse of the sputtering time in the Auger electron spectroscopy, $Io$ decreases, whereas $Ii$ increases. By reducing the sputtering time from the surface of the iron powder to a depth at which ($Io/Ii$) is 0.05 into a thickness, the thickness of the iron oxide film can be calculated.

The "amount of wustite" as referred to herein is an amount expressed by % in terms of an X-ray peak intensity ratio to iron according to the following expression from an integrated intensity of peaks of a (110) plane of iron ($\alpha$Fe) and an integrated intensity of peaks of a (220) plane of FeO (wustite) by using an X-ray diffraction device.

$$[\text{Amount of wustite (\%)}] = 100 \times K_{FeO}/(K\alpha Fe)$$

$K_{FeO}$: Integrated intensity of peaks of a (220) plane of FeO (wustite)
$K\alpha Fe$: Integrated intensity of peaks of a (110) plane of iron ($\alpha$Fe)

Incidentally, in the case where an iron powder having an iron oxide film is prepared by using a mixture containing substances other than the iron powder (for example, a carbon component, a reaction accelerator, and water), the iron powder may be separated from the mixture after the preparation by a magnet, etc. and used as a sample for the measurement. Besides the heat generating composition, in the case of analyzing a heat generating composition in a heat generating body or a heat generating composition molded body, the heat generating composition or heat generating composition molded body is dispersed in nitrogen-purged ion-exchanged water in a nitrogen atmosphere, and an iron powder is separated by a magnet, dried in a nitrogen atmosphere and then provided as a sample for the measurement.

In the case of measuring the water mobility value of the heat generating composition in the heat generating body and the thickness and amount of wustite of the iron oxide film of iron powder in the mixture or the heat generating composition in the heat generating body, the heat generating composition or mixture may be measured according to the following items.

1) Water mobility value:

The heat generating composition is taken out from the heat generating body and measured according to the foregoing method of measuring a water mobility value.

2) Thickness and amount of wustite of iron oxide film of iron powder:

A measuring sample as prepared by dispersing the heat generating composition, the heat generating composition molded body, the heat generating composition compression molded body or the mixture in nitrogen-purged ion-exchanged water in a nitrogen atmosphere, separating the iron powder using a magnet and drying the iron powder in a nitrogen atmosphere is used.

By using the active iron powder of the invention as an iron powder, exothermic rising properties of the heat generating composition are improved so that it is possible to reduce the amount of a carbon component such as active carbon in the heat generating composition by 10 to 20 % or more. By reducing the amount of addition of the carbon component, it is possible to reduce costs and lower a maximum exothermic temperature. Since the exothermic rising properties can be held by an iron powder having an oxygen-containing film on the surface of iron, exothermic characteristics with the passage of time change from a mountain type to a highland type, whereby the heat generation over a long period of time becomes possible.

Although a mechanism of the excellent exothermic rising properties of the heat generating composition of the invention has not be elucidated in detail, it is assumed that because of a catalytic action of wustite against the oxidation of iron and contact between an oxidizing gas and the components to cause oxidation of the components, in particular, oxidation of an iron powder, an iron oxide film, i.e., an oxygen-containing film, is formed on the surface of the iron powder, and oxygen compounds of iron such as oxides, oxyhydroxides, and hydroxides are formed on the surface of the iron component. Also, irregularities, crevices, and the like are generated due to corrosion. As a result, it is assumed that the iron component has hydrophilicity in its own portion. It is assumed that the oxidized iron component is also adhered on the surface of active carbon, whereby hydrophilicity is imparted or improved on the both to cause coupling or structurization among the components through the mediation of water. Furthermore, the case where magnetite ($Fe_3O_4$) is present in the iron oxide film is preferable because it is excellent in conductivity. The case where hematite ($Fe_2O_3$) is present in the iron oxide film is also preferable because it becomes porous.

That is, in the active iron powder of the invention, not only (1) entire (uniform) corrosion, (2) pitting or crevice corrosion, (3) stress corrosion cracking, or the like is generated, whereby a region where a hydroxyl group or an oxygen coupling part is present in the iron powder is generated, but also irregularities or crevices are generated. For that reason, it is assumed that the iron powder of the invention has hydrophilicity and oxidation catalytic properties in its own portion.

In a heat generating composition prepared by only mixing an iron powder with an iron oxide powder or an iron hydroxide powder, exothermic rising properties and hydrophilicity are not revealed. In a low-temperature oxidation reaction as in the heat generating composition of the invention, it is required that iron and iron oxide are present in a state of close contact with each other. It is assumed that first when the surface of iron is partially oxidized, namely, iron and an oxygen compound of iron are co-present, various excellent characteristics of the invention such as excellent exothermic rising properties, exothermic time holding properties, moldability and resistance to compression can be revealed.

Accordingly, it is assumed that in the heat generating composition of the invention, the water which has been absorbed in the heat generating composition causes hydration against a hydrophilic group in other component due to a bipolar mutual action or hydrogen bond and is also present in the surroundings of a hydrophobic portion so as to have high structural properties, whereby the water works as combined water for some meaning. Besides, there is surplus water in a state called as free water, and it is assumed that by adjusting the amount of water, proper amounts of combined water and surplus water are made present in the heat generating composition, whereby the moldability and exothermic properties can be made compatible with each other.

The heat generating composition of the invention contains, as essential components, an iron powder containing from 20 to 100 % by weight of an active iron powder, a carbon component, a reaction accelerator, a hydrogen formation inhibitor and water. If desired, at least one member selected from additional components consisting of a water retaining agent, a water absorptive polymer, a pH adjusting agent, an aggregate, a fibrous material, a functional substance, a surfactant, an organosilicon compound, a pyroelectric substance, a moisturizer, a fertilizer component, a hydrophobic polymer compound, a heat generating aid, a metal other than iron, a metal oxide other than iron oxide, an acidic substance, and a mixture thereof may be added to the heat generating composition of the invention.

A magnetic body may be further added, if desired. Furthermore, the components constituting the heat generating composition and kinds of the components can be used singly or in combination.

Furthermore, in the heat generating composition of the invention, there is no particular limitation for the compounding ratio thereof. Examples thereof include a heat generating composition containing from 1.0 to 50 parts by weight of a carbon component, from 1.0 to 50 parts by weight of a reaction accelerator, from 0.01 to 12 parts by weight of a hydrogen formation inhibitor, from 0.01 to 10 parts by weight of a water retaining agent, from 0.01 to 20 parts by weight of a water absorptive polymer, from 0.01 to 5 parts by weight of a pH adjusting agent, and from 5 to 60 parts by weight of water, respectively based on 100 parts by weight of an iron powder containing from 20 to 100 % by weight of an active iron powder and having a water mobility value of not more than 50.

In addition, the following components may be added.

That is, examples include a metal other than iron in an amount of from 1.0 to 50 parts by weight; a metal oxide other than iron oxide in an amount of from 1.0 to 50 parts by weight; a surfactant in an amount of from 0.01 to 5 parts by weight; an anti-foaming agent in an amount of from 0.01 to 5 parts by weight; a hydrophobic polymer compound, an aggregate, a fibrous material, a pyroelectric substance, a far infrared ray radiating substance, a minus ion emitting substance, and an organosilicon compound each in an amount of from 0.01 to 10 parts by weight; a water-soluble polymer compound and a flocculant each in an amount of from 0.01 to 3 parts by weight; and a moisturizer, a fertilizer component, and a heat generating aid each in an amount of from 0.01 to 10 parts by weight. Incidentally, the compounding ratio of the magnetic body may be properly determined depending upon the desire. Furthermore, in the case of a reaction mixture or in the case of producing a heat generating composition using a reaction mixture, it is to be noted that a portion of 100 parts by weight of an iron powder containing from 20 to 100 % by weight of the active iron powder is defined to be 100 parts by weight of an iron powder containing less than 20 % by weight of the active iron powder or an iron powder not containing the active iron powder.

The water retaining agent is not limited so far as it is able to retain water. Examples thereof include porous materials derived from plants having high capillary function and hydrophilicity such as wood meal, pulp powder, active carbon, saw dust, cotton cloth having a number of cotton fluffs, short fiber of cotton, paper dust, and vegetable materials, water-containing magnesium silicate based clay minerals such as active clay and zeolite, pearlite, vermiculite, silica based porous substances, coralline stone, and volcanic ash based substances (for example, terraballoon, *shirasu* balloon, and *taisetsu* balloon). In order to increase a water retaining ability and enhance a shape holding ability of such a water retaining agent, the water retaining agent may be subjected to a processing treatment such as baking and/or pulverization.

The water absorptive polymer is not particularly limited so far as it is a resin having a crosslinking structure, capable of being swollen upon absorption of water and having a water absorption magnification of ion-exchanged water of 3 times or more of the dead weight. Furthermore, a water absorptive polymer the surface of which is crosslinked may be employed. Conventionally known water absorptive polymers and commercial products can also be employed. Examples of the water absorptive polymer include poly(meth)acrylic acid crosslinked materials, poly(meth)-acrylic acid salt crosslinked materials, sulfonic group-containing poly(meth)acrylic ester crosslinked materials, polyoxyalkylene group-containing poly(meth)acrylic ester crosslinked materials, poly(meth)acrylamide crosslinked materials, crosslinked materials of a copolymer of a (meth)acrylic acid salt and a (meth)acrylamide, crosslinked materials of a copolymer of a hydroxyalkyl (meth)acrylate and a (meth)acrylic acid salt, polydioxolane crosslinked materials, crosslinked polyethylene oxide, crosslinked polyvinylpyrrolidone, sulfonated polystyrene crosslinked materials, crosslinked polyvinylpyridine, saponification products of a starch-poly (meth) acrylonitrile graft copolymer, starch-poly(meth)acrylic acid (salt) graft crosslinked copolymers, reaction products of polyvinyl alcohol and maleic anhydride (salt), crosslinked polyvinyl alcohol sulfonic acid salts, polyvinyl alcohol-acrylic acid graft copolymers, and polyisobutylene maleic acid (salt) crosslinked polymers. These water absorptive polymers may be used alone or in combination with two or more kinds thereof.

Of these water absorptive polymers, water absorptive polymers having biodegradation properties are not limited so far as they are a biodegradable water absorptive polymer. Examples thereof include polyethylene oxide crosslinked materials, polyvinyl alcohol crosslinked materials, carboxymethyl cellulose crosslinked materials, alginic acid crosslinked materials, starch crosslinked materials, polyamino acid crosslinked materials, and polylactic acid crosslinked materials.

The pH adjusting agent is not limited so far it is able to adjust the pH. Examples thereof include alkali metal weak acid salts and hydroxides and alkaline earth metal weak acid salts and hydroxides such as $Na_2CO_3$, $NaHCO_3$, $Na_3PO_4$, $Na_2HPO_4$, $Na_5P_3O_{10}$, NaOH, KOH, $Ca(OH)_2$, $Mg(OH)_2$, and $Ca_3(PO_4)_2$.

The aggregate is not limited so far as it is useful as a filler and/or is useful for making the heat generating composition porous. Examples thereof include fossilized coral (for example, coral fossil and weathered coral fossil), bamboo charcoal, *bincho* charcoal, silica-alumina powders, silica-magnesia powders, kaolin, crystalline cellulose, colloidal silica, pumice, silica gel, silica powders, mica powders, clays, talc, synthetic resin powders or pellets, foamed synthetic resins such as foamed polyesters or polyurethanes, diatomaceous earth, alumina, and cellulose powder.

Incidentally, it is to be noted that kaolin and crystalline cellulose are not contained in the heat generating composition of the invention but contained only in the case where it is used as an adhesive.

The fibrous material is an inorganic fibrous material and/or an organic fibrous material. Examples thereof include rock wool, glass fibers, carbon fibers, metal fibers, pulps, papers, non-woven fabrics, woven fabrics, natural fibers such as cotton and hemp, regenerated fibers such as rayon, semi-synthetic fibers such as acetates, synthetic fibers, and pulverized products thereof.

The functional substance is not limited so far as it is a substance having any function. Examples thereof include at least one member selected from minus ion emitting substances and far infrared ray radiating substances. The minus ion emitting substance is not limited so far as it emits a minus ion as a result either directly or indirectly, and examples thereof include ferroelectric substances such as tourmaline, fossilized coral, granite, and calcium strontium propionate, and ores containing a radioactive substance such as radium and radon. The far infrared ray radiating substance is not limited so far as it radiates far infrared rays. Examples thereof include ceramics, alumina, zeolite, zirconium, and silica.

The surfactant includes anionic surfactants, cationic surfactants, nonionic surfactants, and ampholytic surfactants. Especially, nonionic surfactants are preferable, and examples thereof include polyoxyethylene alkyl ethers, alkylphenol·ethylene oxide adducts, and higher alcohol phosphoric acid esters.

The organosilicon compound is not limited so far as it is a compound having at least an Si-O-R bond and/or an Si-N-R bond and/or an Si-R bond. The organosilicon compound is in the form of a monomer, a lowly condensed product, a polymer, etc. Examples thereof include organosilane compounds such as methyltriethoxysilane; and dimethylsilicone oil, polyorganosiloxane, or silicone resin compositions containing the same.

The pyroelectric substance is not limited so far as it has pyroelectricity. Examples thereof include tourmaline, hemimorphic ores, and pyroelectric ores. Tourmaline or achroite which is a kind of tourmaline is especially preferable. Examples of the tourmaline include dravite, schorl, and elbaite.

The moisturizer is not limited so far as it is able to hold moisture. Examples thereof include hyaluronic acid, collagen, glycerin, and urea.

The fertilizer component is not limited so far as it is a component containing at least one of three elements of nitrogen, phosphorus and potassium. Examples thereof include a bone powder, urea, ammonium sulfate, calcium perphosphate, potassium chloride, and calcium sulfate.

The hydrophobic polymer compound is not limited so far as it is a polymer compound having a contact angle with water of 40° or more, preferably 50° or more, and more preferably 60° or more in order to improve the draining in the composition. The shape of the hydrophobic polymer compound is not limited, and examples thereof include powdery, particulate, granular, and tablet shapes. Examples of the hydrophobic polymer compound include polyolefins such as polyethylene and polypropylene, polyesters, and polyamides.

Examples of the heat generating aid include metal powders, metal salts, and metal oxides such as Cu, Mn, $CuCl_2$, $FeCl_2$, manganese dioxide, cupric oxide, triiron tetroxide, and mixtures thereof.

The acidic substance may be any of an inorganic acid, an organic acid, or an acidic salt. Examples thereof include hydrochloric acid, sulfuric acid, nitric acid, acetic acid, oxalic acid, citric acid, malic acid, maleic acid, chloroacetic acid, iron chloride, iron sulfate, iron oxalate, iron citrate, aluminum chloride, ammonium chloride, and hypochlorous acid.

A process for producing the heat generating composition of the invention is not limited so far as the heat generating composition ultimately contains the essential components and its iron powder is an iron powder containing from 20 to 100 % by weight of an active iron powder. Examples of the production process include:

1) A process for producing a heat generating composition by containing and mixing the active iron powder as produced by the process as set forth above in any one of 1 to 18 and the essential components other than the iron powder;
2) A process for producing a heat generating composition by using, as an iron powder, the active iron powder as produced by the process as set forth above in any one of 1 to 18 and containing and mixing the essential components other than the iron powder;
3) A process for producing a heat generating composition by containing and mixing other components other than the essential components in the heat generating composition as produced in 1) or 2); and
4) A process for producing a heat generating composition by adjusting the water content of the composition as produced in 1) or 2) and mixing.
Incidentally, a production process of a heat generating composition by contact treating a reaction mixture prepared by adding and mixing the essential components and other components with an oxidizing gas and then adjusting the water content is especially preferable.

The "adjustment of the water content" as referred to herein means that after contact treating the heat generating mixture with an oxidizing gas, water or an aqueous solution of a reaction accelerator is added. Although the amount of addition of water or an aqueous solution of a reaction accelerator is not limited, examples thereof include the addition of a weight corresponding to a reduced weight by the contact treatment and the addition of a weight such that a desired water mobility value is obtained.

Whether or nor the adjustment of the water content is introduced may be properly determined depending upon the utility.

Here, with respect to the state of the reaction mixture at the time of the contact treatment with an oxidizing gas, so far as the iron powder is partially oxidized, any of a standing state, a transfer state and a fluidizing state by stirring, etc. may be employed and properly selected. Furthermore, mixing of the respective components of the reaction mixture, heat generating mixture or heat generating composition and mixing at the time of adjusting the water content may be achieved in an oxidizing gas atmosphere or by blowing an oxidizing gas.

The circumferential temperature at the time of contact of the reaction mixture with an oxidizing gas is not limited so far as an iron oxide film and/or wustite is formed at least on the surface of an iron powder. It is preferably 0 °C or higher, more preferably from 10 to 200 °C, further preferably from 20 to 200 °C, still further preferably from 30 to 150 °C, even further preferably from 45 to 100 °C, and even still further preferably from 50 to 80 °C.

The contact time at the time of contact of the reaction mixture with an oxidizing gas is not particularly limited. It is preferably from 2 seconds to 10 minutes, more preferably from 3 seconds to 10 minutes, further preferably from 5 seconds to 5 minutes, and still further preferably from 5 seconds to 3 minutes.

The treatment temperature with an oxidizing gas is not limited so far as the foregoing circumferential temperature is kept. It is preferably from 0 to 200 °C, more preferably from 10 to 200 °C, and further preferably from 20 to 150 °C. Furthermore, the treatment time is preferably from one second to 30 minutes, more preferably from one second to 10 minutes, and further preferably from 2 seconds to 8 minutes.

As the "oxidizing gas" as referred to herein, any substance may be employed so far as it is gaseous and oxidizing. Examples thereof include an oxygen gas, air, and a mixed gas of an inert gas (for example, a nitrogen gas, an argon gas, and a helium gas) and an oxygen gas. As the mixed gas, it is preferable that it contains 10 % or more of an oxygen gas. Of these, air is preferable. If desired, a catalyst such as platinum, palladium, iridium, and compounds thereof can also be used. In the treatment, a concentration of the foregoing mixture is not particularly limited. The oxidation reaction can be carried out under stirring in an oxidizing gas atmosphere optionally under a pressure and/or upon irradiation of ultrasonic waves. The optimal condition of the oxidation reaction may be properly experimentally determined.

An amount of the oxidizing gas to be used is not limited but may be adjusted depending upon the kind of the oxidizing gas, the kind and particle size of the iron powder, the water content, the treatment temperature, the treatment method, and the like. In the case of an open system, there is no limitation so far as a necessary amount of oxygen can be taken in. In the case where air is used in the system of blowing an oxidizing gas, for example, the amount of air is preferably from 0.01 to 1,000 L/min, more preferably from 0.01 to 100 L/min, and further preferably from 0.1 to 50 L/min per 200 g of the iron powder under one atmosphere. In the case of other oxidizing gas, the amount of the oxidizing gas may be reduced into the concentration of oxygen on the basis of the case of air.

If desired, a peroxide may be added. Examples of the peroxide include hydrogen peroxide and ozone.

Furthermore, a magnet may be used for molding the heat generating composition of the invention. By using a magnet, it becomes possible to easily achieve accommodation of the heat generating composition in a mold and separation of the molded body from the mold, thereby making it easier to mold a heat generating composition molded body. In particular, a fixed magnet can be simply provided.

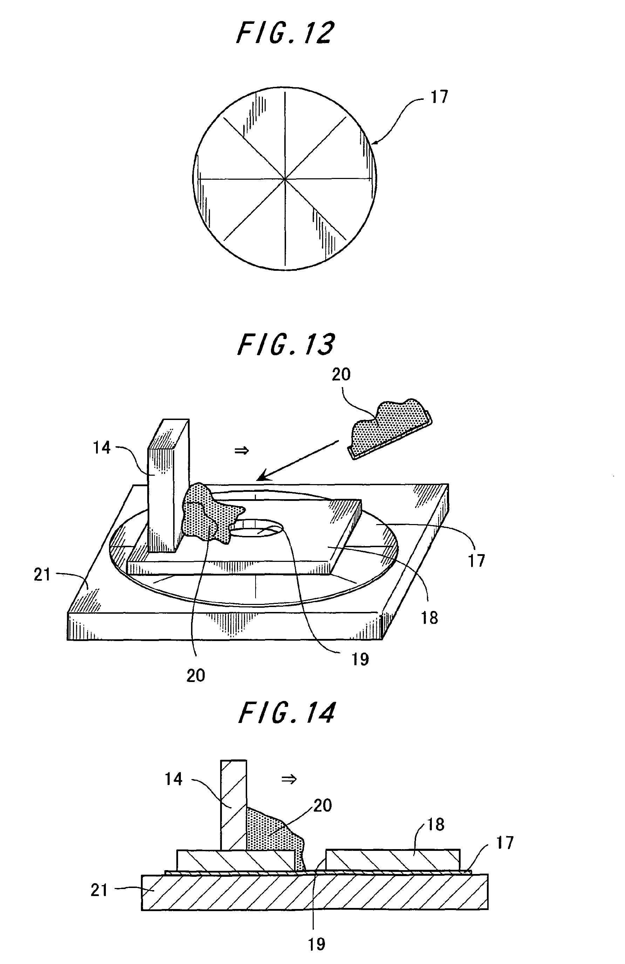

The "water mobility value" as referred to herein is a value showing an amount of surplus water which can transfer to the outside of the heat generating composition in water present in the heat generating composition. This water mobility value will be described below with reference to Figs. 12 to 16.

As shown in Fig. 12, a filter paper 17 of No. 2 (second class of JIS P3801) in which eight lines are drawn radiating from the central point with an interval of 45° is placed on a stainless steel plate 21 as shown in Figs. 13 and 14; a template 18 having a size of 150 mm in length $\times$ 100 mm in width and having a hollow cylindrical hole 19 having a size of 20 mm in inner diameter $\times$ 8 mm in height is placed in the center of the filter paper 17; a sample 20 is placed in the vicinity of the hollow cylindrical hole 19; and a stuffer plate 14 is moved on and along the template 18 and inserted into the hollow cylindrical hole 19 while stuffing the sample 20, thereby leveling the sample (force-in die molding).

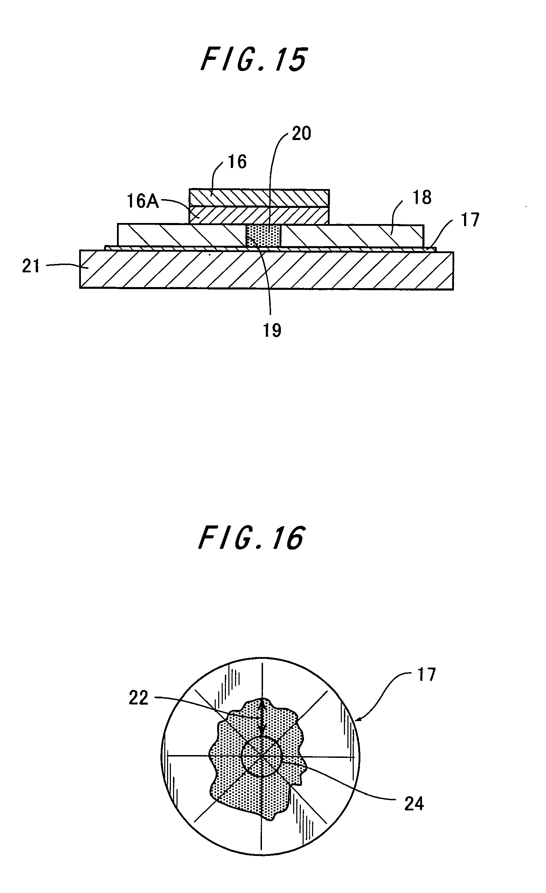

Next, as shown in Fig. 15, a non-water absorptive 70 $\mu$m-thick polyethylene film 16A is placed so as to cover the hole 19, and a flat plate 16 made of stainless steel having a size of 5 mm in thickness $\times$ 150 mm in length $\times$ 150 mm in width is further placed thereon and held for 5 minutes such that an exothermic reaction is not caused.

Thereafter, a shown in Fig. 16, the filter paper 17 is taken out, and an oozed-out locus of the water or aqueous solution is read as a distance 22 (unit: mm) from a periphery 24 as an edge of the hollow cylindrical hole to an oozed-out tip along the radiating lines. Similarly, a distance 22 from each of the lines is read, and eight values in total are obtained.

Each of the eight values (a, b, c, d, e, f, g and h) which are read out is defined as a measured water content value. An arithmetic average value of the eight measured water content values is defined as a water content value (mm) of the sample.

Furthermore, the water content for the purpose of measuring a real water content value is defined as a compounded water content of the heat generating composition corresponding to the weight of the heat generating composition having a size of 20 mm in inner diameter × 8 mm in height or the like, similar measurement is conducted only with water corresponding to that water content, and a value as calculated in the same manner is defined as a real water content value (mm). A value obtained by dividing the water content value by the real water content value and then multiplying with 100 is a water mobility value.

That is, the water mobility value is represented by the following expression.

In the invention, the water mobility value (0 to 100) is preferably from 0.01 to 20, more preferably from 0.01 to 18, further preferably from 0.01 to 15, still further preferably from 0.01 to 13, even further preferably from 1 to 13, and even still further preferably from 3 to 13.

A heat generating composition having a water mobility value of less than 0.01 is insufficient in moldability. A heat generating composition having a water mobility value of from 0.01 to 50 has moldability and therefore, is a moldable heat generating composition. When the water mobility value exceeds 20, it is necessary that a part of water of the heat generating composition is removed by water absorption, dehydration, etc. That is, unless a part of water in the heat generating composition molded body is removed by water absorption, dehydration, etc. using a water absorptive packaging material, etc., a practical useful exothermic reaction is not caused. Incidentally, in the case where a water absorptive polymer having a low water absorption speed is used and although a high water mobility value is exhibited at the time of molding, after elapsing a certain period of time, a part of surplus water is taken in the water absorptive polymer, whereby the heat generating composition becomes in an exothermic state with a water mobility value of from 0.01 to 20, even a heat generating composition having a high water mobility value is dealt as a heat generating composition in which surplus water does not function as a barrier layer. In a heat generating composition having a water mobility value exceeding 50, surplus water is too much, the heat generating composition becomes in a slurry state and loses moldability, and the surplus water functions as a barrier layer. Thus, even upon contact with air as it is, an exothermic reaction is not caused.

Furthermore, the "water mobility value" as referred to herein is a value obtained by digitizing surplus water which is the water content capable of being easily and freely oozed out the system in water which is contained in the heat generating composition or mixture or the like. In a mixture in which some components of the heat generating composition or mixture or the like are mixed, the amount of the surplus water is variously changed depending the amount of a component having a water retaining ability such as a water retaining agent, a carbon component and a water absorptive polymer and wettability of each component, and therefore, it is every difficult to predict the water mobility value from the amount of addition of water. Accordingly, since the amount of surplus water of the heat generating composition or mixture of the like is determined from the water mobility value, by determining the amount of addition of water and the amount of other components, a heat generating composition or mixture or the like having a substantially fixed amount of surplus water is obtained with good reproducibility. That is, by previously examining the water mobility value and a composition ratio of a heat generating composition or mixture or the like, a heat generating composition or mixture or the like as compounded along that composition ratio has a water mobility value falling within a fixed range, namely, an amount of surplus water falling within a fixed range. Thus, it is possible to easily produce a variety of heat generating compositions such as a powdered heat generating composition which causes heat generation upon contact with air but does not have moldability, a heat generating composition which causes heat generation upon contact with air and has moldability, and a heat generating composition which, after discharging out a fixed amount of surplus water from the system by water absorption, etc., causes heat generation upon contact with air and has moldability. Accordingly, if the water mobility value is known, it is possible to note what state does the subject heat generating composition or mixture or the like take.

If the water mobility value is employed, it is possible to embody a desired state with good reproducibility by a simple measurement. Thus, it becomes possible to determine a component ratio of the heat generating composition on the basis of the water mobility value obtained by the measurement and the component ratio, thereby simply achieving actual production of a heat generating composition.

As a use example of the water mobility value, water (or a reaction accelerator aqueous solution) is added to and mixed with a mixture of specified amounts of heat generating composition components exclusive of water (or a reaction accelerator aqueous solution), thereby producing plural heat generating compositions having a different water content. Next, a water mobility value of each of the heat generating compositions is measured, thereby determining a relationship between the amount of addition of water (or a reaction accelerator aqueous solution) and a water mobility value.

A heat generating composition which has moldability and causes heat generation upon contact with air has a water mobility value of from 0.01 to 20. By determining a compounding ratio of the respective components therefrom to prepare a mixture in this compound ratio, a moldable heat generating composition in which water does not function as a barrier layer and which has moldability causes heat generation upon contact with air can be produced with good reproducibility. In this way, since surplus water is used as a connecting substance and a flocculant aid or a dry binding material is not used, reaction efficiency of the iron powder does not drop. Thus, an exothermic performance can be obtained in a small amount as compared with the case of using a flocculant aid or a dry binding material.

Incidentally, in the invention, what water does not function as a barrier layer and causes an exothermic reaction upon contact with air means that water in a heat generating composition does not function as a barrier layer which is an air intercepting layer and immediately after the production of a heat generating composition, comes into contact with air, thereby immediately causing an exothermic reaction.

By using a moldable heat generating composition containing this surplus water as a connecting substance, it becomes possible to produce, for example, a super thin and super flexible heat generating body having plural sectional exothermic parts of a heat generating composition molded body on a substantially planar substrate in a maximum width of preferably from 1 to 50 mm, and more preferably from 1 to 20 mm, or in a maximum diameter of preferably from 1 to 50 mm, and more preferably from 1 to 20 mm (in the case where two or more axes are present as in an ellipse, the major axis is dealt as a length, while the minor axis is dealt as a width).

The "surplus water" as referred to herein means water or an aqueous solution portion which is present excessively in the heat generating composition and easily transfers to the outside of the heat generating composition. The surplus water is defined as a water mobility value which is a value of water or a value of an aqueous solution portion sucked out from the heat generating composition, etc. by a filter paper. When the heat generating composition has an appropriate amount of surplus water, it is assumed that the surplus water causes hydration against hydrophilic groups in the components of the heat generating composition due to a bipolar mutual action or hydrogen bond, etc. and that it is present even in the surroundings of hydrophobic groups while having high structural properties.

This is connecting water as a connecting substance in some meaning. Besides, there is water in a state called as free water which can freely move. When the surplus water increases, the structure is softened, and the free water is found.

The "moldability" as referred to in the invention exhibits that a molded body of the heat generating composition having a cavity or concave die shape is formed by force-through molding using a trimming die having a cavity or cast molding using a concave die, whereby after molding including mold release, the molding shape of the heat generating composition molded body is held.

When the moldability is revealed, since the shape is held until the heat generating composition molded article is at least covered by a covering material and a seal part is formed between the substrate and the covering material, sealing can be achieved in the periphery of the shape with a desired shape. Also, since so-called "spots" which are a collapsed piece of the heat generating composition are not scattered in the seal part, the sealing can be achieved without causing cutting in seal. The presence of the spots causes insufficient sealing.

1) Measurement device:

With respect to the measurement device, a stainless steel-made molding die (a plate having a size of 2 mm in thickness × 200 mm in length × 200 mm in width and having a cavity as treated by R5 in four corners of 60 mm in length × 40 mm in width in a central part thereof) and a fixable leveling plate are disposed above a travelable endless belt, and magnets (two magnets having a size of 12.5 mm in thickness × 24 mm in length × 24 mm in width are disposed in parallel) are disposed under the endless belt.

The magnets should cover a region of the leveling plate and the vicinity thereof and a region larger than a region covered by a cut side (40 mm) vertical to the advancing direction of the cavity of the molding die.

2) Measurement method:

With respect to the measurement method, a stainless steel plate having a size of 1 mm in thickness × 200 mm in length × 200 mm in width is placed on the endless belt of the measurement device, a polyethylene film having a size of 70 μm in thickness × 200 mm in length × 200 mm in width is placed thereon, and a stainless steel-made molding die is further placed thereon.

Thereafter, a leveling plate is fixed in a position of the cavity of the molding die of 50 mm far from the end portion in the advancing direction of the endless belt, 50 g of a heat generating composition is then placed in the vicinity of the leveling plate between the leveling plate and the cavity, and the heat generating composition is filled in the cavity of the molding die while leveling it by moving the endless belt at 1. 8 m/min. After the molding die has completely passed through the leveling plate, the traveling of the endless belt is stopped. Next, the molding die is removed, and a heat generating composition molded body as laminated on the polyethylene film is observed.

3) Judgment method:

With respect to the judgment method, in the surroundings of the heat generating composition molded body, in the case where any collapsed piece of the heat generating composition molded body exceeding a maximum length of 800 μm is not present and the number of collapsed pieces of the heat generating composition molded body having a maximum length of from 300 to 800 μm is not more than 5, it is to be noted that the heat generating composition has moldability.

The moldability is an essential property for a heat generating composition to be used in the molding system. If the heat generating composition does not have moldability, it is impossible to produce a heat generating body by the molding system.

Next, the heat generating body will be described. The heat generating body of the invention is a heat generating body in which a heat generating composition having satisfactory exothermic rising properties is accommodated in an accommodating bag having air permeability in at least a part thereof. In addition, in a preferred heat generating body of the invention, an exothermic part may be formed of one section, or an exothermic part may be formed of a sectional exothermic part in which two or more plural sections are disposed at intervals.

A raw material of the substrate or covering material is not limited so far as it functions as an accommodating bag of the heat generating composition. Usually, raw materials which are used in chemical body warmers or heat generating bodies can be used. Examples of the raw material include air-impermeable raw materials, air-permeable raw materials, water absorptive raw materials, non-water absorptive raw materials, non-extensible raw materials, extensible raw materials, stretchable raw materials, non-stretchable raw materials, foamed raw materials, non-foamed raw materials, non-heat sealable raw materials, and heat sealable raw materials. The raw material can be properly used depending upon a desired utility in a desired form such as films, sheets, non-woven fabrics, woven fabrics, and composites thereof. In general, the substrate is made of an air-impermeable film or sheet, and the covering material is made of an air-permeable film or sheet or non-woven fabric, and vice versa. The both may be air-permeable. As the underlay material, an air-permeable underlay material and an air-impermeable underlay material may be used for different purposes.

The packaging material of the accommodating bag may be of a single-layered structure or multilayered structure, and its structure is not limited. Furthermore, though the packaging material is composed of at least a substrate and a covering material, a packaging material for laminating the heat generating composition molded body is the substrate, and a packaging material for covering on the heat generating composition molded body is the covering material regardless of whether the packaging material is air-permeable or air-impermeable. An embodiment of a multilayered structure in which an air-impermeable packaging material is the substrate and an air-permeable packaging material is the covering material will be hereunder described as one example. That is, in this embodiment, the substrate is made of layer A/layer B, layer A/layer B/layer C, or layer A/layer B/layer C/layer D; and the covering material is made of layer F/layer G, layer E/layer F/layer G, or layer F/layer H/layer G. Examples of the layer A include thermoplastic resin films (for example, polyethylene), heat seal layers (for example, polyethylene and EVA), and water absorptive papers; examples of the layer B include non-woven fabrics of a thermoplastic resin (for example, nylons), non-water absorptive papers, water absorptive papers, thermoplastic resin films (for example, polyethylene films, polypropylene films, polyester films, and polyamide (for example, nylons) films), wicks (for example, non-water absorptive papers and water absorptive papers); examples of the layer C include adhesive layers, non-water absorptive papers, water absorptive papers, thermoplastic resin films (for example, polyethylene), non-slip layers, and non-woven fabrics of a thermoplastic resin (for example, polyesters and nylons); examples of the layer D include separators, thermoplastic resin films (for example, polyethylene), and non-woven fabrics; examples of the layer E include heat seal layers; examples of the layer F include porous films or perforated films made of a thermoplastic resin (for example, polyethylene), films made of a thermoplastic resin (for example, polyethylene), non-water absorptive papers, and water absorptive papers; examples of the layer G include non-woven fabrics of a thermoplastic resin (for example, polyesters and nylons); and examples of the layer H include non-water absorptive papers and water absorptive papers. Examples of the substrate or covering material include heat seal layer made of polyethylene obtained by using a metallocene catalyst/polypropylene film, polyethylene-made heat seal layer/polypropylene film, EVA-made heat seal layer/polypropylene film, EVA-made heat seal layer/polypropylene film/adhesive layer/separator, EVA-made heat seal layer/polyethylene film/nylon non-woven fabric, non-woven fabric/porous film, heat seal layer made of polyethylene obtained by using a metallocene catalyst/polyethylene film/nylon non-woven fabric, heat seal layer made of polyethylene obtained by using a metallocene catalyst/polypropylene film/polypropylene non-woven fabric, non-woven fabric/(paper and/or perforated (provided by a needle or laser) film)/porous film, non-woven fabric/(paper and/or porous film) /perforated (provided by a needle or laser) film, and non-woven fabric/(paper and/or porous film)/non-woven fabric. A method for laminating the respective layers is not limited. The respective layers may be directly laminated; the respective layers may be laminated via an air-permeable adhesive layer or a laminating agent layer; and the respective layers may be laminated by hot melt extrusion or the like. Furthermore, in the invention, it is to be noted that polyethylene produced by using a metallocene catalyst is also included in the polyethylene.

For example, in the case of laminating the foregoing raw material such as non-woven fabrics and porous films via an air-permeable sticky layer, examples of a method for forming the air-permeable sticky layer include a method in which a sticky substance is fibrillated by an appropriate system such as a curtain spray system, a melt blow system or a slot spray system for blowing and spreading a sticky substance via hot air under heat melting and spread and accumulated on an appropriate supporting substrate made of a porous film, an air-permeable substrate, a separator, etc., thereby forming a porous sticky layer.

A thickness of each of the substrate, the covering material, the underlay material, and the raw material constituting the same varies depending upon the utility and is not limited. The thickness is usually from 5 to 5,000 $\mu$m, preferably from 10 to 500 $\mu$m, and more preferably from 20 to 250 $\mu$m.

The air-impermeable raw material is not limited so far as it is air-impermeable. Examples thereof include films, sheets or coatings made of a polymer (for example, polyethylene, polypropylene, nylons, polyacrylates, polyesters, polyvinyl alcohols, and ethylene-vinyl acetate copolymers) and laminates thereof with a metal (including a semiconductor) compound (for example, silicon oxide) or composite raw materials using the same.

Of the foregoing air-impermeable raw materials, examples of a film having high air impermeability include films provided with a single layer or multiple layers of a thin film having a metal including a semiconductor or a compound thereof provided on an air-impermeable raw material film. Examples of the metal including a semiconductor include silicon, aluminum, and alloys or mixtures containing such a metal. Examples of the metal (including a semiconductor) compound include oxides, nitrides and oxynitrides of the foregoing metals or alloys or mixtures. Examples of the layer include silicon oxide layers, aluminum oxide layers, and silicon oxynitride layers; layers obtained by laminating an arbitrary layer of these layers on a polyester-made film; and layers obtained by further laminating a stretched polyolefin film (for example, a biaxially stretched polypropylene film) thereon.

The air-permeable raw material is not limited so far as it is air-permeable. Examples thereof include air-permeable films (for example, porous films and perforated films); materials having air permeability by themselves (for example, papers and non-woven fabrics); materials prepared by laminating at least one of papers and air-permeable films and non-woven fabrics so as to have air permeability; materials prepared by providing an air-impermeable packaging material comprising a non-woven fabric having a polyethylene film laminated thereon with fine pores by using a needle, etc. so as to have air permeability; non-woven fabric whose air permeability is controlled by laminating a fiber and heat bonding under pressure; porous films; and materials prepared by sticking a non-woven fabric onto a porous film. The "perforated film" as referred to herein is a film prepared by providing an air-impermeable film (for example, polyethylene films) with fine pores by using a needle so as to have air permeability.

The air permeability is not limited so far as the heat generation can be kept. In the case of use in usual heat generation, the air permeability is usually from 50 to 10,000 g/m$^2$/24 hr, preferably from 70 to 5,000 g/m$^2$/24 hr, more preferably from 100 to 2, 000 g/m$^2$/24 hr, and further preferably from 100 to 700 g/m$^2$/24 hr in terms of moisture permeability by the Lyssy method.

When the moisture permeability is less 50 g/m$^2$/24 hr, the heat value is small and a sufficient thermal effect is not obtained, and therefore, such is not preferable. On the other hand, when it exceeds 10,000 g/m$^2$/24 hr, the exothermic temperature is high so that a problem in safety may possibly be generated, and therefore, such is not preferable. However, there is no limitation even when the moisture permeability exceeds 10,000 g/m$^2$/24 hr depending upon the utility, or even in the use at a moisture permeability closed to the open system, according to circumstances.

The stretchable packaging material is not particularly limited so far as it is stretchable. That is, it is only required that the stretchable packaging material is stretchable as a whole. The stretchable packaging material may be formed of a single material or a composite material of stretchable substrates or a combination of a stretchable substrate and a non-stretchable substrate.

Examples of the stretchable packaging material include single materials (for example, natural rubbers, regenerated rubbers, synthetic rubbers, elastomers, and stretchable shape memory polymers) and mixtures thereof, mixed materials or blended materials of such a stretchable raw material and a non-stretchable raw material or fabrics constituted of a combination of these materials, films, yarns, strands, ribbons, tapes, and stretchable films with a scrim structure.

The porous film is not limited and can be properly selected among porous films obtained by stretching a film made of a polyolefin based resin (for example, polyethylene, linear low density polyethylene, and polypropylene) or a fluorine based resin (for example, polytetrafluoroethylene) and a filler.

The non-woven fabric is not limited. Single non-woven fabrics of a single fiber or composite fiber made of a material such as rayon, nylons (polyamides), polyesters, polyacrylates, polypropylene, vinylon, polyethylene, polyurethane, cupra, cotton, cellulose, and pulp, or laminates of blended or accumulated fiber layers of such fibers are useful. Furthermore, from the standpoint of production process, dry non-woven fabrics, wet non-woven fabrics, spunbonds, spunlaces, and the like can be used. Non-woven fabrics made of a composite fiber having a core-sheath structure are also useful. A non-woven fabric in the side which is brought into contact with the skin is preferably a napping (fluffy) non-woven fabric. Also, stretchable non-woven fabrics and non-stretchable non-woven fabrics are useful.

The water absorptive raw material is not particularly limited so far as it is a water absorptive film or sheet.

The water absorptive raw material is not particularly limited so far as it has water absorption properties consequently regardless of whether or not the raw material has water absorption properties by itself.

Specific examples thereof include water absorptive foamed films or sheets having water absorption properties (for example, foamed bodies of water absorptive foamed polyurethane, etc.) or papers, non-woven fabrics or woven fabrics formed of a fiber having water absorption properties, non-woven fabrics or woven fabrics containing a fiber having water absorption properties, and water absorptive materials such as water absorptive porous films or sheets. Besides, there are enumerated materials in which regardless of the presence or absence of water absorption properties, a water absorbing agent is contained, impregnated, kneaded, transferred or carried on a foamed film or sheet, a non-woven fabric, a woven fabric or porous film or sheet, thereby imparting or increasing water absorption properties; and materials in which regardless of the presence or absence of water absorption properties, a water absorptive raw material such as water absorptive foamed films or sheets, papers, non-woven fabrics, woven fabrics, and porous films or sheets as cut in a planar shape according to the invention is attached to one side or both sides of the material according to the invention, thereby imparting water absorption properties.

In particular, in the heat generating body of the invention, for the purpose of forming the plane which is brought into contact with the skin into a comfortable plane by imparting water absorption properties against sweat, etc., in order that in the case of sweating, the sweat is absorbed, it is preferable that a packaging material in the plane which is brought into contact with the skin is constituted of a packaging material using a non-woven fabric or a woven fabric containing, as the major component, a water absorptive fiber having a water retention of 20 % or more. Examples of the water absorptive fiber having a water retention of 20 % or more include cottons, silks, hemps, wools, polyacrylonitrile based synthetic fibers, polyamide based synthetic fibers, polyvinyl alcohol based synthetic fibers, acetate fibers, triacetate fibers, and regenerated fibers. In addition, non-woven fabrics having a highly water absorptive polymer held in a non-woven fabric can be used as the non-woven fabric having excellent water absorption properties. Incidentally, non-woven fabrics or woven fabrics containing such a fiber as the major component are relatively good with respect to the feeling against the skin.

In addition, highly water absorptive packaging materials having high absorption properties of sweat can be used as the packaging material. Examples thereof include non-woven fabrics containing a fiber whose surface is coated with a highly water absorptive resin, non-woven fabrics containing a hollow fiber having a number of fine pores on the surface thereof, and non-woven fabrics containing a fiber having a capillary action by forming a number of pouches or plural layers in the cross-sectional shape.

Besides, non-woven fabrics or films having a water absorptive inorganic compound held on a non-sticky surface of a packaging material can be used. Examples thereof include non-woven fabrics resulting from holding a powder (for example, diatomaceous earth, zeolite, and silica gel) on a non-woven fabric and films resulting from holding a relatively large amount of a powder (for example, silica and alumina) on a synthetic resin (for example, polyethylene).

With respect to the exothermic part, the sectional exothermic part and the heat generating composition molded body, any shape may be employed. Examples thereof include a circular shape, a triangular shape, a star shape, a rectangular shape, a square shape, a flower shape, an elliptical shape, a cubic shape, a parallelepiped shape, a polygonal pyramidal shape, a conical shape, a pillar shape, a cylindroid shape, a semi-pillar shape, a semicylindroid shape, a cylindrical shape, and a spherical shape. Furthermore, a concave may be present in the central part or the like of the heat generating composition molded body.

With respect to the exothermic part of the invention, the exothermic part may be formed by one section. Alternatively, sectional exothermic parts may be formed by disposing and fixing two or more plural sections at intervals, and an exothermic part may be formed from a gathering of these sectional exothermic parts and provided for the exothermic part. Furthermore, in the case of an exothermic part having a sectional exothermic part, the size of the heat generating composition molded body on the substrate is not larger than that of the sectional exothermic part, and the periphery of the heat generating composition molded body is heat sealed, thereby constituting the sectional exothermic part and the exothermic part. A capacity of each of the sectional exothermic part and the exothermic part is composed of a capacity of the filling heat generating composition or a capacity of the heat generating composition molded body and a spacial capacity surrounding it (for example, a space surrounded by the covering material and the substrate).

In the sectional exothermic part or the heat generating composition molded body of the invention, its maximum width is usually from 0.5 to 60 mm, preferably from 0.5 to 50 mm, more preferably from 1 to 50 mm, further preferably from 3 to 50 mm, still further preferably 3 to 30 mm, even further preferably from 5 to 20 mm, even still further preferably from 5 to 15 mm, and most preferably from 5 to 10 mm. Furthermore, its maximum height is usually from 0.1 to 30 mm, preferably from 0.1 to 10 mm, more preferably from 0.3 to 10 mm, further preferably from 1 to 10 mm, and still further preferably from 2 to 10 mm. Moreover, its longest length is usually from 5 to 300 mm, preferably from 5 to 200 mm, more preferably from 5 to 100 mm, further preferably from 20 to 150 mm, and still further preferably from 30 to 100 mm.

A capacity of the sectional exothermic part or a volume of the heat generating composition molded body is usually from 0.015 to 500 $cm^3$, preferably from 0.04 to 30 $cm^3$, more preferably from 0.1 to 30 $cm^3$, further preferably from 1 to 30 $cm^3$, and still further preferably from 3 to 20 $cm^3$.

In the sectional exothermic part, when the sectional exothermic part which is an accommodating region of the heat generating composition is filled with the heat generating composition molded body, a volume ratio of the volume of the heat generating composition molded body which is an occupying region of the heat generating composition molded body to the capacity of the sectional exothermic part which is an accommodating region of the heat generating composition is usually from 0.6 to 1, preferably from 0. 7 to 1, more preferably from 0.8 to 1, and further preferably from 0.9 to 1.0.

Furthermore, a width of the sectioned part which is a space between the sectional exothermic parts is not limited so far as sectioning can be achieved. It is usually from 0.1 to 50 mm, preferably from 0.3 to 50 mm, more preferably from 0.3 to 50 mm, further preferably from 0.3 to 40 mm, still further preferably from 0.5 to 30 mm, even further preferably from 1.0 to 20 mm, and even still further preferably from 3 to 10 mm.

In the sectional exothermic part, the accommodating bag, the outer bag (accommodating bag of the heat generating body), and the like, a packaging material or the like which constitutes the same is sealed in the sectioned part and its surroundings. Its seal is not limited but is properly selected depending upon the desire. For example, sealing is carried out in a point-like (missing line) state or entirely by compression seal (adhesive seal), warm compression seal (adhesive seal), bonding seal, heat bonding seal, heat melt seal (heat seal), etc. by means of pressurizing, warming, heating or a combination thereof via an adhesive layer and/or a bonding agent layer and/or a heat seal layer. Selection of any one or a combination of these methods may be made depending upon the desire. In this way, it is possible to seal and form a sectional exothermic part, an inner bag (accommodating bag), an outer bag, etc. Sewing processing can also be employed as one of a seal measure.

In the above, a width of the periphery in a substrate or a substrate for forming an accommodating bag such as a covering material or in the seal part of the sectioned part can be properly determined. It is usually not more than 50 mm, preferably from 1 to 30 mm, and more preferably from 3 to 20 mm.

From the viewpoint of flexibility of the heat generating body, in the case of forming the sectional exothermic part, when its size is small as far as possible, it is possible to make the heat generating body flexible as a whole. In a plan view, it is preferable that one side of a sectional exothermic part which is constituted of at least two sides having a different length from each other is short as far as possible. Also, with respect to a sectional exothermic part which is constituted of the same side as in a square or the like or one diameter as in a circle or the like, it is preferable that the longest length is short as far as possible.

The production process of the heat generating body of the invention is not limited. Examples thereof include a filling system using a filling machine and a molding system using a mold.

That is, the following can be enumerated.

1) A conventional production process by filling a heat generating composition in an air-permeable accommodating bag in a filling system.
This is a process for producing a heat generating body as a continuous formation process in which by using a longitudinal substrate and a rotary heat pressure unit capable of heat sealing a desired partition portion and the periphery of a substrate, while heat sealing the surroundings of the longitudinal substrate and a necessary place of the partition portion as disposed opposite to each other via the heat pressure unit, an air-permeable heat generating body is supplied into a compartment composed of a space between the formed substrates and subjected to a seal treatment; and the formation of a next compartment is initiated while bonding the edge of a body warmer by that seal treatment.
2) A production process of a heat generating body by providing a concave pocket in advance in a substrate by vacuum forming or the like, filling a heat generating composition or a compression molded body thereof in that pocket, further covering a covering material thereon, and then sealing the surroundings of the pocket.
3) A production process of a heat generating body by providing a concave recess on the peripheral surface of a drum-type body of rotation, making a substrate go along that concave recess, filling a heat generating composition on the substrate in the concave recess by magnetism, further covering a covering material thereon, and then sealing the substrate and the covering material in the periphery of the concave recess while fixing the heat generating composition by magnetism.
4) A molding method for laminating a heat generating composition molded body on a longitudinal substrate by molding by filling in a cavity-containing trimming die and transfer into a substrate (force-through molding method).
In the case of a continuous system, this method is a process for producing a heat generating body (continuous force-through molding method) in which a heat generating composition is filled in a cavity of a trimming die by a drum-type body of rotation, etc.; a heat generating molded body in a cavity shape is molded on a longitudinal substrate by using a molding machine capable of achieving molding and lamination, thereby laminating the heat generating composition molded body on the substrate; the laminate is covered by a longitudinal covering material; and by using a rotary seal unit capable of sealing a desired sectioned part and the surroundings of a heat generating body (for example, heat seal, compression seal, and heat compression seal), the desired sectioned part and the surroundings of the heat generating body are sealed via the seal unit and subjected to a seal treatment.
Furthermore, a magnet may be used. By using a magnet, it becomes possible to easily achieve accommodation of the heat generating composition in a mold and separation of the molded body from the mold, thereby making it easier to mold a heat generating composition molded body. In particular, a fixed magnet is simple with respect to installation or maintenance.
5) A molding method for laminating a heat generating composition molded body on a longitudinal substrate by filling in a concave-containing casting mold and transfer into a substrate (cast molding method).

In the case of a continuous system, this method is a process for producing a heat generating body by a cast molding method as a continuous formation method (continuous cast molding method) in which by using a molding machine for laminating a heat generating composition molded body on a longitudinal substrate by filling in a concave, molding and transferring onto a substrate by a drum-type body of rotation, the heat generating composition molded body is molded and laminated on the substrate; the laminate is covered by a longitudinal covering material; and by using a rotary seal unit capable of sealing the periphery of a heat generating composition molded body (for example, heat seal, compression seal, and heat compression seal), the periphery of the heat generating composition molded body is sealed via the seal unit and subjected to a seal treatment.

Furthermore, a magnet may be used. By using a magnet, it becomes possible to easily achieve accommodation of the heat generating composition in a mold and separation of the molded body from the mold, thereby making it easier to mold a heat generating composition molded body.

Furthermore, with respect to the formation of a sectional exothermic part, it can be achieved by magnetic transportation of a specific amount of the heat generating composition into the substrate as described in JP-B-5-81261.

Moreover, in the case of using a pocket-containing substrate, the formation of a sectional exothermic part can be similarly achieved by magnetic transportation of a specific amount of the heat generating composition into the pocket.

Furthermore, as described in JP-T-11-508786, JP-T-2002-514104, and Japanese Patent No. 3164605, the sectional exothermic part can also be prepared by filling a specific amount of the heat generating composition in a pocket between two film layer substrate sheets and sealing it. Moreover, a pocket may be formed by utilizing vacuum. The whole of the descriptions of these patent documents are incorporated herein by reference.

After the accommodation step, a heat generating body is produced through a seal step, a cutting step, and the like. These seal step and cutting step and the like may be properly selected and employed from conventional methods and devices. Incidentally, in the case of a molding system, there are enumerated force-through molding using a trimming die and cast molding using a concave-shaped casting mold.

In the case of a molding system, with respect to the molding order, the size of the heat generating composition molded body is determined, and the size of the sectional exothermic part is then determined.

The particle size of the water-insoluble solid component constituting the moldable heat generating composition of the invention is not limited so far as the heat generating composition has moldability. In the case where any one of length, width and height as the size of the heat generating composition molded body as molded from the heat generating composition is small, the moldability is improved by making the particle size small.

In addition, it is preferable in view of molding that the particle size of the solid component constituting the moldable heat generating composition is small. A maximum particle size of the water-insoluble solid component exclusive of the reaction accelerator and water in the components constituting the moldable heat generating composition is preferably not more than 2.5 mm, more preferably not more than 930 $\mu$m, further preferably not more than 500 $\mu$m, still further preferably not more than 300 $\mu$m, even further preferably not more than 250 $\mu$m, and even still further preferably not more than 200 $\mu$m. Moreover, 80 % or more of the particle size of the solid component is usually not more than 500 $\mu$m, preferably not more than 300 $\mu$m, more preferably not more than 250 $\mu$m, further preferably not more than 200 $\mu$m, still further preferably not more than 150 $\mu$m, and even further preferably not more than 100 $\mu$m.

Incidentally, with respect to the particle size of the water-insoluble solid component, separation is conducted using a sieve, and the particle size of the component which has passed through the sieve is calculated from an opening of the sieve. That is, sieves of 8, 12, 20, 32, 42, 60, 80, 100, 115, 150, 200, 250 and 280 meshes and a receiving dish are combined in this order from up to down. About 50 g of water-insoluble solid component particles are placed on the uppermost 8-mesh sieve and shaken for one minute using an automatic shaker. Weights of the water-insoluble solid component particles on each of the sieves and the receiving dish are weighed. The total amount thereof is defined as 100 %, and the particle size distribution is determined from weight fractions. When the sum of all receiving dishes under the sieve of a specific mesh size becomes 100 % which is the total sum of the particle size distribution, the size ($\mu$m) calculated from the opening of the specific mesh is defined as the particle size of the water-insoluble solid component. Incidentally, each of the mesh sieves may be combined with other mesh sieves. Here, the particles which have passed through a 16-mesh sieve are defined to have a particle size of not more than 1 mm; the particles which have passed through a 20-mesh sieve are defined to have a particle size of not more than 850 $\mu$m; the particles which have passed through a 48-mesh sieve are defined to have a particle size of not more than 300 $\mu$m; the particles which have passed through a 60-mesh sieve are defined to have a particle size of not more than 250 $\mu$m; the particles which have passed through a 65-mesh sieve are defined to have a particle size of not more than 200 $\mu$m; the particles which have passed through an 80-mesh sieve are defined to have a particle size of not more than 180 $\mu$m; the particles which have passed through a 100-mesh sieve are defined to have a particle size of not more than 150 $\mu$m; the particles which have passed through a 115-mesh sieve are defined to have a particle size of not more than 120 $\mu$m; the particles which have passed through a 150-mesh sieve are defined to have a particle size of not more than 100 $\mu$m; and the particles which have passed through a 250-mesh sieve are defined to have a particle size of not more 63 $\mu$m, respectively. The same is applicable to mesh sizes of less than these mesh sizes.

Furthermore, the heat generating composition can be classified into a powder, a granulate heat generating composition (having a water mobility value of less than 0.01), a moldable heat generating composition (having a water mobility value of from 0.01 to 20), and a sherbet-like heat generating composition (having a water mobility value exceeding 20 but not more than 50) depending upon the state of adjustment of the water content or surplus water. The heat generating composition as classified depending upon the water mobility value is as described previously.

Of the substrate, the covering material, the air-permeable adhesive layer, the underlay material, and the heat generating composition including the heat generating composition molded body, at least the heat generating composition may be subjected to a compression treatment. In particular, in a material prepared by properly compressing the heat generating composition molded body of the invention by pressurization, its shape holding characteristic is markedly improved. For example, even when a perforated film which is difficult with respect to the pressure adjustment is used as a raw material of the air-permeable part in place of the porous film, or even when an inner pressure of the accommodating bag becomes equal to or more than the outer pressure, shape collapse hardly occurs so that the use of a perforated film is possible. Accordingly, not only the range for selecting an air-permeable raw material is widened so that the costs can be lowered, but also a body to be warmed can be uniformly warmed at an appropriate temperature over a long period of time.

A heat generating body in which a number of sectional exothermic parts are continuously provided at intervals and the perforation is provided to a degree such that cutting by hand is possible in the sectioned exothermic part can be cut into an appropriate size at the time of use on the basis of the purpose for use adaptive with a place for application of a human body, or the like and applied. In that case, the size of the heat generating body and the size and number of the sectional exothermic parts may be properly set up. There are no limitations regarding such size and number. Furthermore, the sectioned part can be formed in arbitrary directions such as a length or width direction, length and width directions, and an oblique direction.

The "perforation" as referred to in the invention includes one which is intermittently cut for the purpose of improving flexural properties of the sectioned part and one which is intermittently cut such that cutting by hand is possible. Its degree, length and aperture are not limited but are determined depending upon the desire. The perforation may be provided in all sectioned parts or may be partially provided. The shape is not particularly limited, and examples thereof include a circle, an ellipse, a rectangle, a square, and a cut line (linear shape). For example, in the perforation which is intermittently cut such that cutting by hand is possible, a circular hole having an aperture of from $\phi$10 to 1,200 $\mu$m can be enumerated. The aperture of the hole is more preferably from $\phi$20 to 500 $\mu$m.

It is preferable that the holes are positioned lined up in the length and width. Furthermore, a shortest space between outer peripheries of the adjacent holes in the length and width is not limited so far as it is satisfactory with flexural properties and possibility of cutting by hand. The shortest space is preferably from 10 to 2,000 $\mu$m, more preferably from 10 to 1,500 $\mu$m, further preferably from 20 to 1,000 $\mu$m, still further preferably from 20 to 500 $\mu$m, and even further preferably from 20 to 200 $\mu$m. The cutting properties by hand are remarkably improved by a balance between the aperture of the hole and the shortest space of outer peripheries of the adjacent holes in the length and width.

The hole may be a cut line, and its length may be a length corresponding to the aperture or may be larger than the aperture. A shortest space between ends of the adjacent cut lines in the length and width is corresponding to the shortest space between outer peripheries of the adjacent holes.

For example, an aperture of the hole of from $\phi$10 to 2, 000 $\mu$m is corresponding to a length of from 10 to 2,000 $\mu$m, and a shortest space between outer peripheries of the adjacent holes in the length and width of from 10 to 2,000 $\mu$m is corresponding to a shortest space between ends of the adjacent cut lines in the length and width of from 10 to 2,000 $\mu$m. In the case of a cut line, since it becomes long in one direction, its length can be prolonged and may be from 10 to 50,000 $\mu$m.

The fixing means is not limited so far as it has capability for fixing a thermal packaging body for joint surroundings or a material having an exothermic part to a prescribed part.

As the fixing means, an adhesive layer, a hook and eye, a hook and button, a hook and loop fastener such as Velcro, a magnet, a band, a string, and combination thereof can be arbitrarily used.

Incidentally, in the case of a band, fixing means for adjustment may be further constructed by a combination of a hook and loop fastener and an adhesive layer.

Here, the "hook and loop fastener" as referred to herein has a fastening function by a combination of a loop as a female fastener with a male fastener capable of fastening the female fastener thereto, which is known as trade names such as Magic Tape (a registered trademark), Magic Fastener (a registered trademark), Velcro Fastener, and Hook and Loop Tape. Examples of the material having a loop function include non-woven fabrics and woven fabrics of napped or hole-containing yarns. Such a material having a loop function (female fastener function) may be covered on the surface of a paddling forming the band, or the band may be constructed of such a material itself. Although the hook member which is the male fastener member is not particularly limited, examples thereof include hook members formed of a polyolefin based resin (for example, polyethylene and polypropylene), a polyamide, a polyester, etc. Although the shape of the hook is not particularly limited, a hook having a cross-sectional shape such as an I type, an inverted L type, an inverted J type, and a so-called mushroom type is preferable because it is easily hooked by the loop and does not give an extreme stimulus to the skin. Incidentally, the hook may be adhered to the entire area of a fastening tape, and only the hook may be used as a fastening tape while omitting a tape substrate.

The adhesive layer may contain at least one member selected from additional components consisting of a water retaining agent, a water absorptive polymer, a pH adjusting agent, a surfactant, an organosilicon compound, a hydrophobic polymer compound, a pyroelectric substance, an antioxidant, an aggregate, a fibrous material, a moisturizer, a functional substance, and a mixture thereof.

The adhesive of the invention is classified into a non-hydrophilic adhesive, a mixed adhesive, and a hydrophilic adhesive (for example, a gel).

The adhesive constituting the adhesive layer is not limited so far as it has an adhesive strength necessary for adhering to the skin or clothes. Adhesives of every form such as a solvent based adhesive, an aqueous adhesive, an emulsion type adhesive, a hot melt type adhesive, a reactive adhesive, a pressure-sensitive adhesive, a non-hydrophilic adhesive, and a hydrophilic adhesive are employable.

The adhesive layer includes one layer of a non-hydrophilic adhesive constituted of the non-hydrophilic adhesive and non-hydrophilic adhesive layers constituted of the non-hydrophilic adhesive.

It is to be noted that a material whose water absorption properties are improving by containing a water absorptive polymer or a water retaining agent in the non-hydrophilic adhesive layer is dealt as the non-hydrophilic adhesive layer.

A hot melt based adhesive may be provided between the hydrophilic adhesive layer and a substrate or a covering material. Furthermore, in the case where the hydrophilic adhesive is provided in a thermal packaging body for joint surroundings, there is no limitation. After seal treating a thermal packaging body for joint surroundings, a hydrophilic adhesive layer may be provided in the thermal packaging body for joint surroundings.

Furthermore, the adhesive layer may or may not have air permeability and may be properly selected depending upon the utility. With respect to the air permeability, the adhesive layer may be air-permeable as a whole. Examples thereof include an adhesive layer having air permeability as a whole of a region in which an adhesive is partially present and a portion where no adhesive is present is partially present.

In laminating an adhesive on an air-permeable substrate and/or a covering material in a stratiform state as it is, examples of a method for keeping its air permeability include a method in which an adhesive layer is partially laminated by printing or transferring an adhesive, thereby forming a non-laminated part as an air-permeable part; a method in which an adhesive is transferred in one direction while drawing a circle in a filament-like form or properly moved in the two-dimensional directions by transferring in a zigzag manner, whereby a space of the filament-like adhesive keeps air permeability or moisture permeability or the adhesive is foamed; and a method for forming a layer by a melt blow system.

Examples of the adhesive which constitutes the non-hydrophilic adhesive layer include acrylic adhesives, polyvinyl acetate based adhesives (for example, vinyl acetate resin based emulsions and ethylene-vinyl acetate resin based holt melt adhesives), polyvinyl alcohol based adhesives, polyvinyl acetal based adhesives, vinyl chloride based adhesives, polyamide based adhesives, polyethylene based adhesives, cellulose based adhesives, chloroprene (neoprene) based adhesives, nitrile rubber based adhesives, polysulfide based adhesives, butyl rubber based adhesives, silicone rubber based adhesives, styrene based adhesives (for example, styrene based hot melt adhesives), rubber based adhesives, and silicone based adhesives. Of these, rubber based adhesives, acrylic adhesives, and adhesives containing a hot melt based polymer substance for the reasons that they are high in the adhesive strength, are cheap, are good in long-term stability, and are small in reduction of the adhesive strength even by providing heat.

In addition to the base polymer, if desired, the adhesive may be compounded with other components such as tackifiers (for example, petroleum resins represented by rosins, chroman-indene resins, hydrogenated petroleum resins, maleic anhydride-modified rosins, rosin derivatives, and C-5 based petroleum resins), phenol based tackifiers (especially, tackifiers having an aniline point of not higher than 50 °C; for example, terpene phenol based resins, rosin phenol based resins, and alkylphenol based resins), softeners (for example, coconut oil, castor oil, olive oil, camellia oil, and liquid paraffin), softeners, anti-aging agents, fillers, aggregates, adhesion adjusting agents, adhesion modifiers, coloring agents, anti-foaming agents, thickeners, and modifiers, thereby improving performance such as an improvement in adhesion to nylon-made clothes and mixed yarn clothes.

Examples of the hot melt based adhesive include known hot melt based adhesives imparted with adhesion. Specific examples thereof include styrene based adhesives made of, as a base polymer, an A-B-A type block copolymer (for example, SIS, SBS, SEBS, and SIPS), vinyl chloride based adhesives made of, as a base polymer, a vinyl chloride resin, polyester based adhesives made of, as a base polymer, a polyester, polyamide based adhesives made of, as a base polymer, a polyamide, acrylic adhesives made of, as a base polymer, an acrylic resin, polyolefin based adhesives made of, as a base polymer, a polyolefin (for example, polyethylene, super low density polyethylene, polypropylene, ethylene-$\alpha$-olefin copolymers, and ethylene-vinyl acetate copolymers), 1,2-polybutadiene based adhesives made of, as a base polymer, 1,2-polybutadiene, and polyurethane based adhesives made of, as a base polymer, polyurethane;

adhesives made of a modified body of the foregoing adhesive whose adhesion is improved or whose stability is changed; and mixtures of two or more kinds of these adhesives. Adhesive layers constituted of a foamed adhesive and adhesive layers constituted of a crosslinked adhesive can also be employed.

The non-aromatic hot melt based adhesive is not limited so far as it is made of, as a base polymer, a hot melt based adhesive not containing an aromatic ring. Examples thereof include olefin based hot melt based adhesives and acrylic hot melt based adhesives. As the non-aromatic polymer which is the base polymer not containing an aromatic ring, there are enumerated polymers or copolymers of an olefin or a diene. Examples thereof include olefin polymers. The olefin polymer includes polymers or copolymers of ethylene or an $\alpha$-olefin. Also, polymers resulting from adding a diene (for example, butadiene and isoprene) as other monomer thereto may be employed.

The $\alpha$-olefin is not limited so far as it is a monomer having a double bond in the terminal thereof. Examples thereof include propylene, butene, heptane, hexene, and octene.

The "aromatic hot melt based adhesive" as referred to herein is a hot melt based adhesive whose base polymer contains an aromatic ring. Examples thereof include styrene based hot melt based adhesives represented by A-B-A type block copolymers.

In the foregoing A-B-A type block copolymers, the A block is a non-elastic polymer block made of a monovinyl substituted aromatic compound A such as styrene and methylstyrene; and the B block is an elastic polymer block made of a conjugated diene such as butadiene and isoprene. Specific examples thereof include a styrene-butadiene-styrene block copolymer (SBS), a styrene-isoprene-styrene block copolymer (SIS), and hydrogenated types thereof (for example, SEBS and SIPS), and mixtures thereof.

As a countermeasure for preventing a lowering of adhesive strength caused due to an increase of water of the non-hydrophilic adhesive layer, an adhesive layer obtained by further compounding a water absorptive polymer in the non-hydrophilic adhesive can be used.

The hydrophilic adhesive which constitutes the hydrophilic adhesive layer is not particularly limited so far as it contains a hydrophilic polymer or a water-soluble polymer as the major component, has adhesion and is hydrophilic as an adhesive.

Examples of the constitutional components of the hydrophilic adhesive include hydrophilic polymers (for example, polyacrylic acid), water-soluble polymers (for example, poly(sodium acrylate) and polyvinylpyrrolidone), crosslinking agents (for example, dry aluminum hydroxide and meta-silicic acid aluminic acid metal salts), softeners (for example, glycerin and propylene glycol), higher hydrocarbons (for example, soft liquid paraffin and polybutene), primary alcohol fatty acid esters (for example, isopropyl myristate), silicon-containing compounds (for example, silicone oil), fatty acid glycerin esters (for example monoglycerides), oily components (for example, vegetable oils such as olive oil), antiseptics (for example, methyl p-hydroxybenzoate and propyl p-hydroxybenzoate), solubilizing agents (for example, N-methyl-2-pyrrolidone), thickeners (for example, carboxymethyl cellulose), surfactants (for example, polyoxyethylene hardened castor oil and sorbitan fatty acid esters), hydroxycarboxylic acid (for example, tartaric acid), excipients (for example, light silicic anhydride, water absorptive polymers, and kaolin), moisturizers (for example, D-sorbitol), stabilizers (for example, sodium edetate, p-hydroxybenzoic acid esters, and tartaric acid), crosslinking type water absorptive polymers, boron compounds (for example, boric acid), and water. They may be used as an arbitrary combination.

A temporary adhering seal part is formed via a sticky layer. An adhesive which constitutes the sticky layer is a layer formed of a polymer composition which is tacky at the normal temperature and is not limited so far as it can be heat sealed after temporary adhesion.

Furthermore, the foregoing adhesives of the sticky layer can be used as the adhesive which constitutes the sticky layer as used for temporary adhesion. Of these, non-hydrophilic adhesives are preferable. With respect to the adhesive constituting the adhesive layer, it is preferable that the adhesive is well compatible with a heat seal material constituting a heat seal and that a melting point of the base polymer of the adhesive is not higher than a melting point of the heat seal material. Hot melt based adhesives are especially preferable for hot melt based bonding agents. Furthermore, in the case where the heat seal material is an olefin based raw material, preferred examples thereof include olefin based adhesives.

A bonding layer for fixing the air permeability adjusting material is constituted of a bonding agent or an adhesive which is usually used. In particular, an adhesive is useful, and the foregoing adhesives for constituting the adhesive layer can be used.

Furthermore, a method for providing a bonding layer is not limited so far as the air permeability adjusting material can be fixed. The bonding layer may be entirely provided or partially or intermittently provided. Examples of its shape include various shapes such as a network-like shape, a stripe-like shape, a dot-like shape, and strip-like shape.

Furthermore, in the case where an adhesive layer is employed as the hydrophilic adhesive layer, if there is a difference in a water retaining force between the hydrophilic adhesive layer and the heat generating composition molded body, transfer of water occurs via a packaging material present therebetween such as a substrate, thereby causing in-conveniences against the both. In particular, the transfer of water occurs during the storage. In order to prevent this, it is preferable that the packaging material present therebetween at least has a moisture permeability of not more than 2 $g/m^2/day$ in terms of a moisture permeability according to the Lyssy method. By using this, in the case where the heat generating body is accommodated in an outer bag as an air-impermeable accommodating bag and stored, the transfer of water can be prevented.

In the case where a hydrophilic adhesive layer is used as the adhesive layer, the moisture permeability of a moisture-proof packaging material provided between the heat generating composition molded body and the hydrophilic adhesive layer is not limited so far as the transfer of water can be prevented within the range where the exothermic performance is not affected. The moisture permeability according to the Lyssy method is usually not more than 2 $g/m^2$/day, preferably not more than 1.0 $g/m^2$/day, more preferably not more than 0.5 $g/m^2$/day, and further preferably from 0.01 to 0.5 $g/m^2$/day. These values are a value under a condition under an atmospheric pressure at 40 °C and 90 % RH. Incidentally, the moisture-proof packaging material can be used as a substrate or a covering material and may be laminated singly on a substrate, a covering material, or the like.

The moisture-proof packaging material is not limited so far as the transfer of water between the heat generating composition molded body and the hydrophilic adhesive layer can be prevented. Examples thereof include metal vapor deposited films, vapor deposited films of a metal oxide, metal foil-laminated films, EVOH (ethylene/vinyl alcohol copolymer or ethylene/vinyl acetate copolymer saponified product) based films, biaxially stretched polyvinyl alcohol films, polyvinylidene chloride coated films, polyvinylidene chloride coated films obtained by coating polyvinylidene chloride on a substrate film (for example, polypropylene), metal foils such as an aluminum foil, air-impermeable packaging materials obtained by vapor depositing or sputtering a metal (for example, aluminum) on a polyester film substrate, and packaging laminates using a transparent barrier film of a structure in which silicon oxide or aluminum oxide is provided on a flexible plastic substrate. The air-impermeable packaging materials which are used in the outer bag, etc. can also be used.

Furthermore, packaging materials such as moisture-proof packaging materials as described in JP-A-2002-200108, the disclosures of which can be incorporated herein by reference, can be used.

In the case of using a water-containing hydrophilic adhesive (for example, a gel) in the adhesive layer, in order to adjust the moisture equilibrium between the heat generating composition and the adhesive layer, the content of a reaction accelerator (for example, sodium chloride) or a substance having a water holding power (for example, a water absorptive polymer) in the heat generating composition may be adjusted within the range of from 10 to 40 % by weight, preferably from 15 to 40 % by weight, and more preferably from 15 to 30 % by weight based on the heat generating composition. Furthermore, as the adhesive having good moisture permeability and low stimulation to the skin, water-containing adhesives (for example, hydrophilic adhesives and gels) as described in JP-A-10-265373 and JP-A-9-87173, adhesives which can be subjected to hot melt coating as described in JP-A-6-145050 and JP-A-6-199660, and rubber based adhesives as described JP-A-10-279466 and JP-A-10-182408, the disclosures of which are totally incorporated herein by reference, are useful.

The functional substance which is contained in the adhesive layer is not limited so far as it is a substance having any function. There can be enumerated at least one member selected from aromatic compounds, vegetable extracts, crude drugs, perfumes, slimming agents, analgesics, blood circulation promoters, swelling improvers, antibacterial agents, sterilizers, mold inhibitors, odor eaters, deodorants, percutaneously absorptive drugs, fat-splitting components, minus ion generators, far infrared ray radiants, magnetic bodies, fomentations, cosmetics, bamboo vinegar, and wood vinegar. Specific examples thereof include aromatic compounds (for example, menthol and benzaldehyde), vegetable extracts (for example, mugwort extract), crude drugs (for example, moxa), perfumes (for example, lavender and rosemary), slimming agents (for example, aminophylline and tea extract), analgesic drugs (for example, indomethacin and dl-camphor), blood circulation promoters (for example, acidic mucopolysaccharide and chamomile), swelling improvers (for example, horse chestnut extract and flavone derivatives), fomentations (for example, aqueous boric acid, physiological saline, and aqueous alcohols), fat-splitting components (for example, jujube extract, caffeine, and tonalin), cosmetics (for example, aloe extracts, vitamin preparations, hormone preparations, anti-histamines, and amino acids), antibacterial agents and sterilizers (for example, carbolic acid derivatives, boric acid, iodine preparations, invert soaps, salicylic acid based substances, sulfur, and antibiotics), and mold inhibitors.

The percutaneously absorptive drug is not particularly limited so far as it has percutaneous absorption. Examples thereof include corticosteroids, anti-inflammatory drugs, hypertension drugs, anesthetics, hypnotic sedatives, tranquillizers, antibacterial substances, antifungal substances, skin stimulants, inflammation inhibitors, anti-epileptics, analgesics, antipyretics, anesthetics, mold inhibitors, antimicrobial antibiotics, vitamins, antiviral agents, swelling improvers, diuretics, antihypertensives, coronary vasodilators, anti-tussive expectorants, slimming agents, anti-histamines, antiarrhythmic agents, cardiotonics, adrenocortical hormones, blood circulation promoters, local anesthetics, fat-splitting components, and mixtures thereof.

However, it should not be construed that the invention is limited thereto. These drugs are used singly or in admixture of two or more kinds thereof as the need arises.

The content of such a functional substance is not particularly limited so far as it falls within the range where the effect of a medicine can be expected. However, from the viewpoints of adhesive strength as well as pharmacological effect and economy, the content of the functional substance is preferably from 0.01 to 25 parts by weight, and more preferably from 0.5 to 15 parts by weight based on 100 parts by weight of the adhesive.

Furthermore, a method for providing the adhesive layer is not limited so far as a thermal packaging body for joint surroundings can be fixed. The adhesive layer may be entirely provided or partially or intermittently provided. Examples of its shape include various shapes such as a network-like shape, a stripe-like shape, a dot-like shape, and strip-like shape.

The heat generating body may be accommodated in an outer bag which is an air-impermeable accommodating bag, stored and transported. The outer bag is not limited so far as it is air-impermeable and may be made of a laminate. Examples thereof include air-impermeable raw materials such as nylon, polyester and polypropylene films which are subjected to a moisture-proof treatment with OPP, CPP, polyvinylidene chloride, metal oxides (including semiconductors) such as aluminum oxide and silicon oxide, etc., aluminum foils, and aluminum-deposited plastic films.

Furthermore, among the foregoing air-impermeable raw materials, examples of a film having high air impermeability include films in which a single layer or multiple layers made of a metal including semiconductors or a compound thereof are provided on an air-impermeable raw material film. Examples of the metal including semiconductors include silicon, aluminum, titanium, tin, indium, and alloys or mixtures of these metals.

Examples of the metal compound including semiconductors include oxides, nitrides and oxynitrides of the foregoing metals or alloys or mixtures.

Examples of the layer include a silicon oxide layer, an aluminum oxide layer, a silicon oxynitride layer, and layers obtained by laminating arbitrary layers thereof.

Furthermore, there are also numerated layers obtained by laminating a stretched polyolefin film (for example, a biaxially stretched polypropylene film) on the foregoing layer.

Examples of the heat generating body which is accommodated in an outer bag include a heat generating body in which a produced heat generating body is sealed between two air-impermeable films or sheets.

At least one member or a part of the substrate, the covering material, the adhesive layer and the separator, all of which constitute the heat generating body, may be provided with at least one kind of characters, designs, symbols, numerals, patterns, photographs, pictures, and colored parts.

Each of the substrate, the covering material, the adhesive layer and the separator, all of which constitute the heat generating body, may be transparent, opaque, colored, colorless, or the like. Furthermore, the layer which constitutes at least one layer of the layers constituting the respective materials and layers may have a colored part as colored different from other layers.

The heat generating composition which can be used for the heat generating body of the invention includes a powder or granulate heat generating composition (having a water mobility value of less than 0.01), a moldable heat generating composition (having a water mobility value of from 0.01 to 20), and a sherbet-like heat generating composition (having a water mobility value of from 20 to 50). With respect to a production process, there is no limitation. Usually, in accordance with a powder or granulate heat generating composition having a water mobility value of less than 0.01, a heat generating body is produced by a filling system or a magnetic fixing system; in accordance with a heat generating composition having a water mobility value of from 0.01 to 20, a heat generating body is produced by a molding system, a filling system, or a magnetic fixing system; and in accordance with a heat generating composition having a water mobility value exceeding 20 but not more than 50, a heat generating body is produced by a molding system.

Furthermore, at least one member of the heat generating composition molded body, the substrate, the covering material, and the underlay material may be entirely or partly subjected to a pressurizing treatment or provided with irregularities. In this may, the transfer of the layered body between the substrate and the covering material may be prevented.

The heat generating composition of the invention has resistance to compression. The "resistance to compression" as referred to herein means that a heat generating composition compressed body obtained by compressing a heat generating composition molded body as accommodated in a molding die within the die to such an extent that the thickness is 70 % of the die thickness holds 80 % or more of exothermic rising properties of the exothermic rising properties of the heat generating composition molded body before compression (a difference in temperature between one minute and 3 minutes after starting a heat generation test of the heat generating composition).

Here, the measurement method of exothermic rinsing properties for the resistance to compression will be described below.

  1. Heat generating composition molded body:

      1) A magnet is provided in the vicinity of a central part of the back side of a polyvinyl chloride-made supporting plate (3 mm in thickness $\times$ 600 mm in length $\times$ 600 mm in width) of a footed supporting table so as to cover a cavity shape of a molding die.
      2) A temperature sensor is placed on the central part the surface of the supporting plate.
      3) A polyethylene film (25 $\mu$m in thickness $\times$ 250 mm in length $\times$ 200 mm in width) as provided with an adhesive layer having a thickness of about 80 $\mu$m is stuck onto the supporting plate via a sticky layer such that the center of the polyethylene film is positioned at the sensor.
      4) On an underlay plate (280 mm in length $\times$ 150 mm in width $\times$ 50 $\mu$m to 2 mm in thickness), a polyethylene film (230 mm in length × 155 mm in width × 25 μm to 100 μm in thickness) is placed such that one end of the polyethylene film is projected by about 20 mm outside the underlay plate and that one end thereof in the length direction is substantially coincident with one end of the underlay plate.

5) A template (230 mm in length × 120 mm in width × 3 mm in thickness) having a cavity (80 mm in length × 50 mm in width × 3 mm in height) is placed on the polyethylen film placed on the underlay plate; a template is placed on the polyethylene film such that one end thereof in the length direction is fitted to one end where the underlay plate and the polyethylene film are coincident with each other and that in the width direction, one end part of the width of the template is placed at a position of the central part by about 20 mm far from an opposing end to the side where the polyethylene film is projected outward from the underlay plate. Next, the resulting assembly is placed on the supporting plate together with the underlay plate.

6) A sample is placed in the vicinity of the cavity; a force-in die plate is moved along the molding die; the sample is charged into the cavity while stuffing; and the sample is leveled while stuffing along the template plane (force-in die molding), thereby filling the sample in the die.

7) Next, the magnet beneath the supporting plate is removed; the end portion of the projected polyethylene film is pressed; the underlay plate is removed; and the temperature measurement is started.

2. Heat generating composition compressed body:

1) to 6) are the same as in the case of the heat generating composition molded body.

8) A die having a convex having a thickness of 0.9 mm which can substantially tightly come into the cavity in relation of the cavity with an unevenness is fitted to the cavity and compressed by a roll press or plate press to prepare a heat generating composition compressed body having a thickness of 2.1 mm (compressed to 70 % of the die thickness) within the die.

9) The resulting assembly is placed on the supporting plate together with the underlay plate; the magnet beneath the supporting plate is removed; the end portion of the projected polyethylene film is pressed; the underlay plate is removed; and the temperature measurement is started.

With respect to the measurement of the exothermic temperature, the temperature is measured for 5 minutes at a measurement timing of 2 seconds using a data collector, and resistance to compression is judged in terms of a difference in temperature between after elapsing one minute and after elapsing 3 minutes.

The thickness after compression is preferably from 50 to 99.5 %, more preferably from 60 to 99.5 %, and further preferably from 60 to 95 % of the die thickness.

Incidentally, in the invention, it is to be noted that the heat generating composition molded body includes a heat generating composition compressed body.

A method for measuring a temperature rise of the heat generating composition is as follows.

1) A heat generating composition is allowed to stand in a state that it is sealed in an air-impermeable outer bag for one hour under a condition that the circumferential temperature is 20 ± 1 °C.

2) A magnet is provided in the vicinity of a central part of the back side of a polyvinyl chloride-made supporting plate (5 mm in thickness × 600 mm in length × 600 mm in width) of a footed supporting table so as to cover a cavity shape of a molding die.

3) A temperature sensor is placed on the central part of the supporting plate.

4) A polyethylene film (25 μm in thickness × 250 mm in length × 200 mm in width) as provided with an adhesive layer having a thickness of about 80 μm is stuck onto the supporting plate via a sticky layer such that the center of the polyethylene film is positioned at the sensor.

5) The heat generating composition is taken out from the outer bag.

6) A template (250 mm in length × 200 mm in width) having a cavity (80 mm in length × 50 mm in width × 3 mm in height) is placed above the central part of the polyethylene film; a sample is placed in the vicinity of the cavity; a force-in die plate is moved along the template; the sample is charged into the cavity while stuffing; and the sample is leveled while stuffing along the template plane (force-in die molding), thereby filling the sample in the die. Next, the magnet beneath the supporting plate is removed, and the temperature measurement is started.

With respect to the measurement of the exothermic temperature, the temperature is measured for 10 minutes at a measurement timing of 2 seconds using a data collector, and exothermic rising properties are judged in terms of the temperature after elapsing 3 minutes.

The heat generation test of the heat generating body follows the JIS temperature characteristic test.

The heat generating body of the invention is able to give various shapes, thicknesses and temperature zones and therefore, can be used for various utilities such as use for a joint, facial esthetic use, use for eyes, slimming use, use for heating or warming a dripping solution, use for a wet compress pack, use for a medical body warmer, use for a neck, use for a waist, use for a mask, use for a glove, use for hemorrhage, use for relaxation of symptoms such as shoulder pain, muscular pain, and menstrual pain, use for a cushion, use for heating or warming a human body during the operation, use for a thermal sheet, use for thermally volatilizing an aroma, use for an abdomen, insecticidal use by thermal volatilization, and use for treating cancer in addition to common warming of a human body. In addition, the heat generating body of the invention can be used for heating or warming machines, pets, etc.

For example, in the case of using for relaxation of symptoms, the heat generating body of the invention is applied directly in a necessary site of the body or indirectly via a cloth, etc. Furthermore, in the case of using for heating or warming a human body during the operation, a method for using the heat generating body of the invention includes the following methods.

(1) The heat generating body is directly applied to a body requiring heating or warming.
(2) The heat generating body is fixed on a covering, etc. and covered on the body.
(3) The heat generating body is fixed on a cushion to be placed beneath the body, etc.
(4) The heat generating body is used as a covering or a cushion which is a product having the heat generating body provided therein in advance.
Incidentally, examples of the pain of muscles or bones include acute muscle pain, acute bone pain, acute reference pain, previous muscle pain, previous bone pain, chronic reference pain, and join pain of knee, elbow, etc.
The holding time is not limited but is preferably from 20 seconds to 24 hours, more preferably from one hour to 24 hours, and further preferably from 8 hours to 24 hours.
The holding temperature is preferably from 30 to 50 °C, more preferably from 32 to 50 °C, further preferably from 32 to 43 °C, still further preferably from 32 to 41 °C, and even further preferably from 32 to 39 °C.

Next, the invention will be specifically described with reference to the Examples, but it should not be construed that the invention is limited thereto.

[Brief Description of the Drawings]

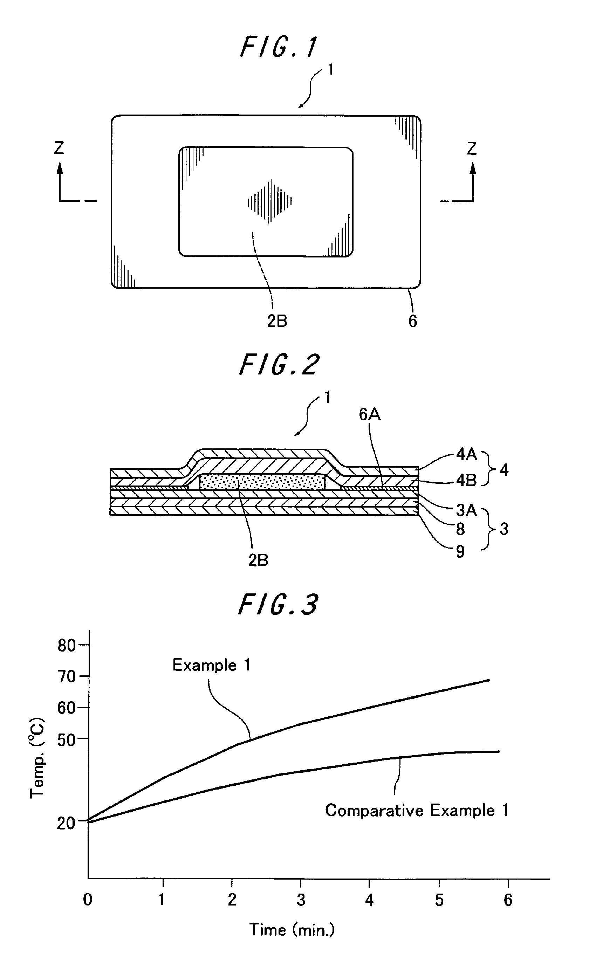

[Fig. 1] is a plan view of an embodiment of the heat generating body of the invention.
[Fig. 2] is a cross-sectional view along the line Z-Z of the same.
[Fig. 3] is a diagram of exothermic characteristics of Example 1 and Example 2.

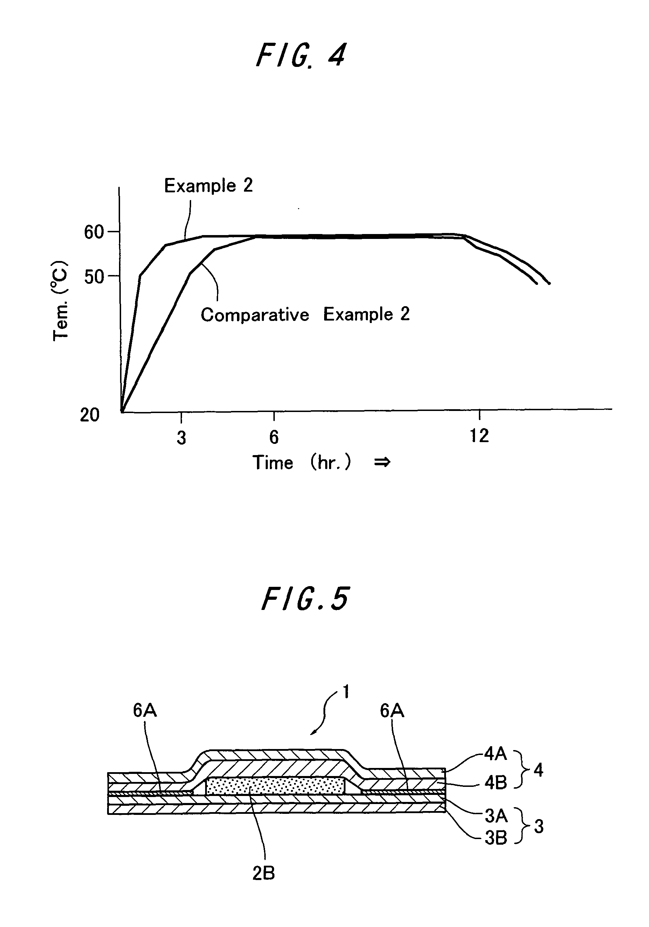

[Fig. 4] is a diagram of exothermic characteristics of Example 2 and Example 3.
[Fig. 5] is a cross-sectional view of another embodiment of the heat generating body of the invention.

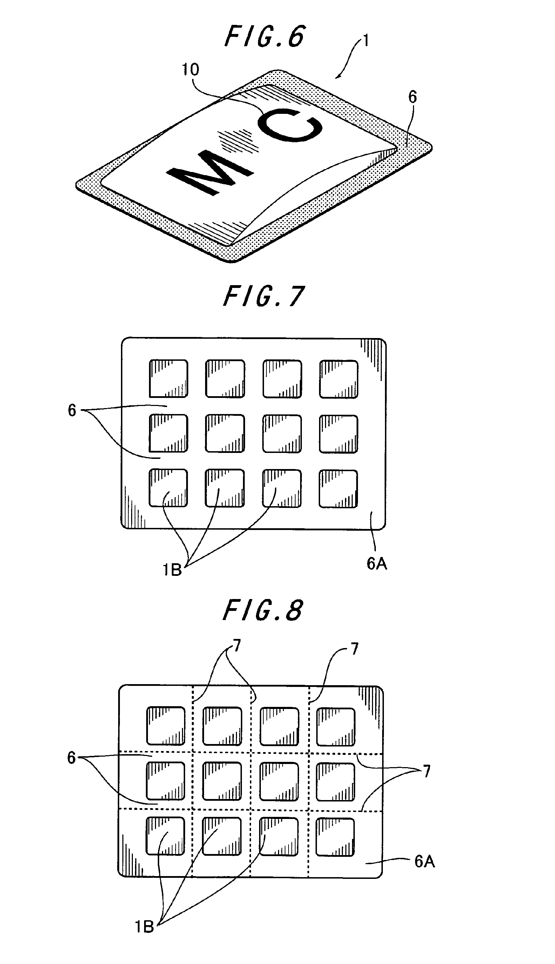

[Fig. 6] is an oblique view of another embodiment of the heat generating body of the invention.
[Fig. 7] is a plan view of another embodiment of the heat generating body of the invention.
[Fig. 8] is a plan view of a still another embodiment of the heat generating body of the invention.

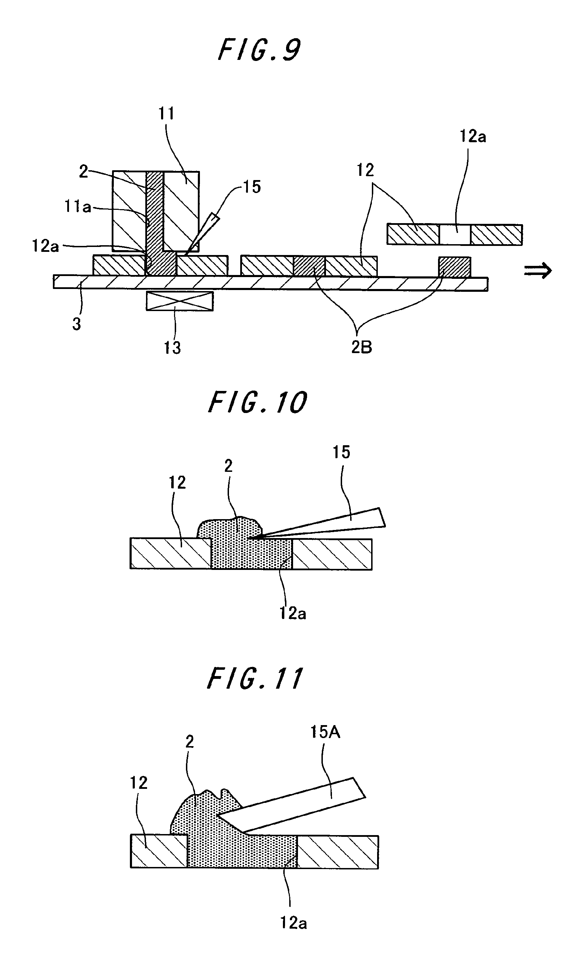

[Fig. 9] is a schematic view of force-through molding of the heat generating body of the invention by using a leveling plate.
[Fig. 10] is an explanatory view of the leveling plate of the same.
[Fig. 11] is an explanatory view of a pushing leveling plate of the same.
[Fig. 12] is a plan view of a filter paper for the measurement of water mobility value in the invention.
[Fig. 13] is an oblique view for explaining the measurement of water mobility value in the invention.
[Fig. 14] is a cross-sectional view for explaining the measurement of water mobility value in the invention.
[Fig. 15] is a cross-sectional view for explaining the measurement of water mobility value in the invention.
[Fig. 16] is a plan view of a filter paper after carrying out the measurement of water mobility value in the invention.

[Description of Reference Numerals and Signs]

1:      Heat generating body
2B:    Heat generating composition molded body
3:      Substrate
4:      Covering material
9:      Separator

10:         Design
11:         Die
11a:        Die hole
12:         Mold
12a:        Mold hole
13:         Magnet
14:         Pushing plate
15:         Leveling plate
15A:        Pushing leveling plate
16:         Flat plate
16A:        Non-water absorptive film (polyethylene film, etc.)
17:         Filter paper in which eight lines are drawn radiating from the central point with an interval of 45°
18:         Die plate
19:         Hole
20:         Sample
21:         Stainless steel plate
22:         Distance to the oozed-out locus of water or aqueous solution
24:         Position corresponding to a hollow cylindrical hole on filter paper

[Examples]

(Example 1)

A stirring type batchwise oxidizing gas contact treatment device consisting of a mixer equipped with a rotary blade for stirring was used as an oxidizing gas contact treatment device, and air was used as an oxidizing gas. First of all, a reaction mixture consisting of 100 parts by weight of a reduced iron powder (particle size: not more than 300 $\mu$m), 5.2 parts by weight of active carbon (particle size: not more than 300 $\mu$m), 0.7 parts by weight of sodium sulfite, and 10 parts by weight of 11 % salt water and having a water mobility value of less than 0.01 was charged in the stirring type batchwise oxidizing gas contact treatment device.
Next, in the state that the upper portion of the oxidizing gas contact treatment device was opened to air, the reaction mixture was subjected to self heat generation with stirring under circumstances at 20 °C and contact treated with an oxidizing gas at a maximum exothermic temperature of 55 °C until the exothermic temperature reached 35 °C, thereby obtaining a contact treated reaction mixture. With respect to the iron powder of the contact treated reaction mixture, a thickness of the resulting iron oxide film on the surface of the iron powder was measured by the Auger electron spectroscopy. The thickness of the iron oxide film was 100 nm. Next, 11 % salt water was mixed in the contact treated reaction mixture to obtain a heat generating composition having a water mobility value of 10.

(Comparative Example 1)

A heat generating composition having a water mobility value of 10 was prepared in the same manner as in Example 1, except that the contact treatment with an oxidizing gas was not carried out.
Each of the heat generating compositions as obtained in Example 1 and Comparative Example 1 was subjected to an exothermic test, thereby obtaining the results as shown in Fig. 3. Comparative Example 1 was deteriorated in exothermic rising properties, whereas the heat generating composition of Example 1 was excellent in exothermic rising properties.

(Example 2)

A stirring type batchwise oxidizing gas contact treatment device consisting of a mixer equipped with a rotary blade for stirring was used as an oxidizing gas contact treatment device, and air was used as an oxidizing gas. First of all, a reaction mixture consisting of 100 parts by weight of a reduced iron powder (particle size: not more than 300 $\mu$m), 5.2 parts by weight of active carbon (particle size: not more than 300 $\mu$m), 2. 3 parts by weight of a wood meal (particle size: not more than 300 $\mu$m), 2.3 parts by weight of a water absorptive polymer (particle size: not more than 300 $\mu$m), 0.2 parts by weight of calcium hydroxide, 0.7 parts by weight of sodium sulfite, and 10 parts by weight of 11 % salt water and having a water mobility value of less than 0.01 was charged in the stirring type batchwise oxidizing gas contact treatment device vessel.
Next, in the state that the upper portion of the oxidizing gas contact treatment device vessel was opened to air, the reaction mixture was subjected to self heat generation with stirring under circumstances at 25 °C and contact treated with an oxidizing gas at a maximum exothermic temperature of 68 °C until the exothermic temperature reached 35 °C, thereby obtaining a contact treated reaction mixture. With respect to the iron powder of the contact treated reaction mixture, a thickness of the resulting iron oxide film on the surface of the iron powder was measured by the Auger electron spectroscopy. The thickness of the iron oxide film was 200 nm. Next, 11 % salt water was mixed in the contact treated reaction mixture to obtain a heat generating composition having a water mobility value of 8.

This heat generating composition was subjected to an exothermic test of heat generating composition. As a result, the temperature reached about 50 °C (an average value of five samples) after 3 minutes.

Furthermore, the heat generating composition was tested for moldability. As a result, even after separating a trimming die from a heat generating composition molded body, the heat generating composition molded body was free from a loss of shape, and collapsed pieces of the heat generating composition molded body were not generated in the surroundings of the heat generating composition molded body.

Next, as illustrated in Figs. 1 and 2, a heat generating composition molded body 2B which was prepared by force-through molding by using a trimming die having a rectangular cavity of 2 mm in thickness, 110 mm in length and 80 mm in width was laminated on a polyethylene film 3A of an air-impermeable substrate 3 provided with a separator 9 on the polyethylene film 3A via an adhesive layer 8. In addition, an air-permeable covering material 4 made of a laminate of a nylon-made non-woven fabric 4A and a porous film 4B was superimposed thereon such that the surface of the polyethylene film 3A and the surface of the porous film 4B were brought into contact with each other. The surroundings were heat sealed in a seal width of 8 mm and then cut to prepare a rectangular flat heat generating body 1 of 130 mm in length, 100 mm in width and 8 mm in seal width. Even after separating the trimming die from the heat generating composition molded body 2B, the laminate was free from a loss of shape, and collapsed pieces of the heat generating composition molded body were not generated in the surroundings of the heat generating composition molded body. Also, sealing could be completely carried out without causing incorporation of collapsed pieces of the heat generating composition molded body into the seal part, and seal failure did not occur. Incidentally, the air permeability of the covering material 4 was 370 g/m$^2$/24 hr in terms of a moisture permeability by the Lyssy method.

Next, the heat generating body was sealed and accommodated in an air-impermeable outer bag and then allowed to stand at room temperature for 24 hours.

After 24 hours, the heat generating body was taken out from the outer bag and subjected to an exothermic test by the body. As a result, it was felt warm after 3 minutes, and thereafter, the warmth was continued for 10 hours or more. Fig. 5 shows an example in which the substrate 3 of the heat generating body 1 of Fig. 2 is replaced by a substrate made of polyethylene film 3A/non-woven fabric 3B. Fig. 6 shows an example in which characters 10 are provided on one surface of the heat generating body 1.

(Comparative Example 2)

A heat generating composition was obtained in the same manner as in Example 2, except that the contact treatment with an oxidizing gas was not carried out, from which was then obtained a heat generating body. The heat generating body was subjected to an exothermic test by a human body in the same manner as in Example 2. As a result, it took 6 minutes until it was felt warm.

With respect to Example 2 and Comparative Example 2, the exothermic test of heat generating body was carried out. As a result, as shown in Fig. 4, in the case of Example 2, the temperature was 50 °C after 30 minutes and 58 °C after 3 hours, respectively. However, in the case of Comparative Example 2, the temperature was 28 °C after 30 minutes and 44 °C after 3 hours, respectively. The heat generating body using the heat generating composition of the invention was excellent in exothermic rising properties.

(Example 3)

100 parts by weight of an iron powder having a wustite content of 11 % by weight (particle size: not more than 300 μm), 5.3 parts by weight of active carbon (particle size: not more than 300 μm), 5 parts by weight of a wood meal (particle size: not more than 300 μm), 1.2 parts by weight of a water absorptive polymer (particle size: not more than 300 μm), 0.2 parts by weight of calcium hydroxide, 0.7 parts by weight of sodium sulfite, and 11 % salt water were mixed to obtain a heat generating composition having a water mobility value of 5.

Furthermore, the heat generating composition was tested for moldability. As a result, even after separating a trimming die from a heat generating composition molded body, the heat generating composition molded body was free from a loss of shape, and collapsed pieces of the heat generating composition molded body were not generated in the surroundings of the heat generating composition molded body.

Next, a heat generating composition molded body as molded by means of force-through molding by using a trimming die having a rectangular cavity of 2 mm in thickness, 110 mm in length and 80 mm in width was laminated on a polyethylene film of an air-impermeable substrate in which an adhesive layer provided with a separator was provided on the polyethylene film. In addition, an air-permeable covering material made of a laminate of a nylon-made non-woven fabric and a porous film was superimposed thereon such that the surface of the polyethylene film and the surface of the porous film were brought into contact with each other. The surroundings were heat sealed in a seal width of 8 mm and then cut to prepare a rectangular flat heat generating body of 130 mm in length, 100 mm in width and 8 mm in seal width. Even after separating the trimming die from the heat generating composition molded body, the laminate was free from a loss of shape, and collapsed pieces of the heat generating composition molded body were not generated in the surroundings of the heat generating composition molded body. Also, sealing could be completely carried out without causing incorporation of collapsed pieces of the heat generating composition molded body into the seal part, and seal failure did not occur. Incidentally, the air permeability of the covering material was 380 g/m$^2$/24 hr in terms of a moisture permeability by the Lyssy method.

Next, the heat generating body was sealed and accommodated in an air-impermeable outer bag and then allowed to stand at room temperature for 24 hours.

After 24 hours, the heat generating body was taken out from the outer bag and subjected to an exothermic test by the body. As a result, it was felt warm within 3 minutes, and thereafter, the warmth was continued for 10 hours or more.

(Example 4)

In the same manner as in Example 3, an exothermic reaction mixture consisting of 100 parts by weight of an active iron powder-free iron powder (particle size: not more than 300 $\mu$m), 3.4 parts by weight of active carbon (particle size: not more than 300 $\mu$m), 3 parts by weight of a water absorptive polymer (particle size: not more than 300 $\mu$m), 0.5 parts by weight of calcium hydroxide, 0.7 parts by weight of sodium sulfite, and 8 parts by weight of 11 % salt water and having a water mobility value of less than 0.01 was charged in the contact treatment device vessel. Next, in the state that the upper portion of the contact treatment device vessel was opened to air, the reaction mixture was subjected to self heat generation with stirring and contact treated with an oxidizing gas at a maximum exothermic temperature of 67 °C until the exothermic temperature reached 35 °C, thereby obtaining the exothermic reaction mixture which had been subjected to a contact treatment with an oxidizing gas. The thickness of the iron oxide film of the iron powder in the exothermic reaction mixture which had been subjected to a contact treatment with an oxidizing gas was 200 nm. 11 % salt water was mixed to obtain a heat generating composition having a water mobility value of 5.

This heat generating composition was subjected to an exothermic test of heat generating composition. As a result, the temperature reached about 50 °C (an average value of five samples) after 3 minutes.

Furthermore, the heat generating composition was tested for moldability. As a result, even after separating a trimming die from a heat generating composition molded body, the heat generating composition molded body was free from a loss of shape, and collapsed pieces of the heat generating composition molded body were not generated in the surroundings of the heat generating composition molded body.

Next, a rectangular flat heat generating body of 130 mm in length, 100 mm in width and 8 mm in seal width was prepared in the same manner as in Example 3.

Next, the heat generating body was sealed and accommodated in an air-impermeable outer bag and then allowed to stand at room temperature for 24 hours.

After 24 hours, the heat generating body was taken out from the outer bag and subjected to an exothermic test. As a result, it was felt warm within 3 minutes, and thereafter, the warmth was continued for 10 hours or more.

(Comparative Example 3)

A heat generating composition having a water mobility value of 8 was obtained by using the components excluding sodium sulfite from the heat generating composition of Example 3. The resulting heat generating body was then sealed and accommodated in an air-impermeable outer bag in the same manner as in Example 3.

The heat generating bodies, each of which had been prepared and accommodated in an air-impermeable outer bag in Example 4 and Comparative Example 3, respectively, were each stored at 60 °C for 30 days, thereby measuring swelling of the outer bag. The swelling of the heat generating body of Example 4 was 3 % and fell within a practically useful range, whereas the swelling of the heat generating body of Comparative Example 4 was very large as 30 % and was not practically useful. From these results, it was understood that a combination of an iron powder having a large wustite amount with a hydrogen formation inhibitor enabled one to provide a heat generating body which is suppressed with respect to the amount of formation of a gas, is free from swelling of the outer bag, is excellent in exothermic rising properties, and has exothermic holding properties.

(Example 5)

In the same manner as in Example 3, an exothermic reaction mixture consisting of 100 parts by weight of an iron powder having a wustite content of less than 1 % by weight (particle size: not more than 300 $\mu$m), 5. 3 parts by weight of active carbon (particle size: not more than 300 $\mu$m), 3 parts by weight of a water absorptive polymer (particle size: not more than 300 $\mu$m), 0.5 parts by weight of calcium hydroxide, 0.7 parts by weight of sodium sulfite, and 8 parts by weight of 11 % salt water and having a water mobility value of less than 0.01 was charged in the contact treatment device vessel. Next, in the state that the upper portion of the contact treatment device vessel was opened to air, the reaction mixture was subjected to self heat generation with stirring under circumstances at 20 °C and contact treated with an oxidizing gas at a maximum exothermic temperature of 58 °C until the exothermic temperature reached 35 °C, thereby obtaining the exothermic reaction mixture which had been subjected to a contact treatment with an oxidizing gas. The thickness of the iron oxide film of the iron powder in the exothermic reaction mixture which had been subjected to a contact treatment with an oxidizing gas was 100 nm. 11 % salt water was added to the mixture which had been subjected to a contact treatment with an oxidizing gas, followed by adjusting the water content and mixing to obtain a heat generating composition having a water mobility value of 8. By using, as a substrate, an air-impermeable packaging material having a polyethylene film laminated on a non-woven fabric, the heat generating composition was molded and laminated on the polyethylene by force-through molding using a trimming die having a rectangular cavity of 2 mm in thickness, 110 mm in length and 80 mm in width, thereby obtaining a heat generating composition molded body. In addition, an air-permeable packaging material made of a laminate of a nylon-made non-woven fabric and a porous film in this order was used as a covering material and superimposed thereon such that the surface of the polyethylene and the surface of the porous film were brought into contact with each other. The surroundings were heat sealed and then cut to prepare a rectangular flat heat generating body of 130 mm in length, 100 mm in width and 8 mm in seal width. Even after separating the trimming die from the heat generating composition molded body, the laminate was free from a loss of shape, and collapsed pieces of the heat generating composition molded body were not generated in the surroundings of the heat generating composition molded body. Also, sealing could be completely carried out without causing incorporation of collapsed pieces of the heat generating composition molded body into the seal part, and seal failure did not occur. Incidentally, the air permeability of the covering material was 370 g/m$^2$/24 hr in terms of a moisture permeability by the Lyssy method.

Next, the heat generating body was sealed and accommodated in an air-impermeable outer bag and then allowed to stand at room temperature for 24 hours. After 24 hours, the heat generating body was taken out from the outer bag and subjected to an exothermic test. As a result, the temperature reached 34 °C within 3 minutes, and an exothermic duration of 34 °C or higher was 8 hours. Furthermore, the heat generating body was sealed and accommodated in an air-impermeable outer bag was stored at 60 °C for 30 days, thereby measuring swelling of the outer bag. As a result, the swelling was 2 % and exhibited practical usefulness as a heat generating body.

(Example 6)

In the same manner as in Example 5, a heat generating composition molded body of 2 mm in thickness, 110 mm in length and 80 mm in width was provided on a substrate by a force-through molding method using a trimming die, and a covering material was covered thereon. Thereafter, the heat generating composition molded body was compressed so as to have a thickness of 1 mm by roll compression, and the surroundings were sealed and then cut to prepare a heat generating body of 130 mm in length, 100 mm in width and 8 mm in seal width. The heat generating body was sealed and accommodated in an air-impermeable outer bag and then allowed to stand at room temperature for 24 hours. After 24 hours, the heat generating body was taken out from the outer bag and subjected to an exothermic test. As a result, the temperature reached 34 °C within 3. 5 minutes, and an exothermic duration of 34 °C or higher was 8 hours. A lowering in exothermic characteristics due to the compression was not substantially observed.

(Comparative Example 5)

By using a heat generating composition as prepared in the same manner as in the preparation of the heat generating composition prior to carrying out the treatment with an oxidizing gas in Example 6, except for changing the water mobility value to 3 and using an iron powder having a thickness of an iron oxide film of less than 30 nm, a heat generating composition molded body of 2 mm in thickness, 110 mm in length and 80 mm in width was provided on a substrate by a force-through molding method using a trimming die, and a covering material was then covered thereon. Thereafter, the heat generating composition molded body was compressed so as to have a thickness of 1 mm by roll compression, and the surroundings were sealed and then cut to prepare a heat generating body having a seal width of 8 mm. The heat generating body was sealed and accommodated in an air-impermeable outer bag and then allowed to stand at room temperature for 24 hours in the same manner as in Example 1. After 24 hours, the heat generating body was taken out from the outer bag and subjected to an exothermic test. As a result, in order that the temperature reached 34 °C, it took 10 minutes, and an exothermic duration of 34 °C or higher was 3 hours. A lowering in exothermic characteristics due to the compression was observed.

(Example 7)

20 g of a heat generating composition the same as in Example 5 was charged and sealed in a non-water absorptive air-permeable accommodating bag to prepare a heat generating body of 130 mm in length, 100 mm in width and 8 mm in seal width. Incidentally, the air permeability of the covering material was 370 g/m$^2$/24 hr in terms of a moisture permeability by the Lyssy method. The heat generating body was sealed and accommodated in an air-impermeable outer bag and then allowed to stand at room temperature for 24 hours. After 24 hours, the heat generating body was taken out from the outer bag and subjected to an exothermic test by the body. As a result, it was felt warm within 3 minutes, and thereafter, the warmth was continued for 10 hours or more.

(Example 8)

By using a heat generating composition as obtained in the same manner as in Example 3, 12 square heat generating composition molded bodies having a thickness of 2 mm and a length of one side of 25 mm were molded by force-through molding using a trimming die having a thickness of 2 mm and composed of 9 square cavities having a length of one side of 15 mm and laminated on a substrate, and an air-permeable covering material was then covered thereon. The surroundings of each of the square heat generating composition molded bodies were heat sealed (6) in a seal width 5 mm and a surrounding seal part of an accommodating bag was heat sealed (6A) in a seal width 8 mm, thereby preparing a gathered exothermic part heat generating body 1 made of 12 sectional exothermic parts 1B having an external dimension of 131 mm × 101 mm (see Fig. 8). Incidentally, the air permeability of the covering material was 370 g/m$^2$/24 hr in terms of a moisture permeability by the Lyssy method. The heat generating body 1 was sealed and accommodated in an air-impermeable outer bag and allowed to stand at room temperature for 24 hours in the same manner as in Example 3. After 24 hours, the heat generating body was taken out from the outer bag and subjected to an exothermic test by the body. As a result, it was felt warm within 3 minutes, and thereafter, the warmth was continued for 10 hours or more. Flexibility of the heat generating body was kept before, during and after the use. An adhesive strength of the adhesive layer beneath the sectioned seal part 6 of the present heat generating body was weaker than that of the adhesive layer beneath the sectional exothermic part 1B. Incidentally, Fig. 7 is an example of seal wherein the seal part is plain, and Fig. 8 is a modified example thereof wherein a perforation 7 from which cutting by handing is possible is provided in the seal part.

(Example 9)

An iron powder consisting of 67 parts by weight of an iron powder having a thickness of an iron oxide film 300 nm and 33 parts by weight of an iron powder having a wustite amount of less than 1 % by weight, 6 parts by weight of active carbon (particle size: not more than 300 μm), 5 parts by weight of a wood meal (particle size: not more than 300 μm), 4 parts by weight of a water absorptive polymer (particle size: not more than 300 μm), 0.3 parts by weight of calcium hydroxide, 1 part by weight of sodium sulfite, and 11 % salt water were mixed to obtain a heat generating composition having a water mobility value of 10.

The heat generating composition was tested for moldability. As a result, even after separating a trimming die from a heat generating composition molded body, the heat generating composition molded body was free from a loss of shape, and collapsed pieces of the heat generating composition molded body were not generated in the surroundings of the heat generating composition molded body. Furthermore, this heat generating composition was subjected to an exothermic test. As a result of measuring the temperature, the temperature reached about 60 °C (an average value of five samples) after 10 minutes.

Next, by using, as a substrate, an air-impermeable packaging material having a polyethylene film laminated on a non-woven fabric, the heat generating composition was molded and laminated on the polyethylene by force-through molding using a trimming die having a rectangular cavity of 2 mm in thickness, 120 mm in length and 84 mm in width, thereby obtaining a heat generating composition molded body. In addition, an air-permeable packaging material made of a laminate of a nylon-made non-woven fabric and a porous film in this order was used as a covering material and superimposed thereon such that the surface of the polyethylene and the surface of the porous film were brought into contact with each other. The surroundings were heat sealed and then cut to prepare a rectangular flat heat generating body 1 of 136 mm in length, 100 mm in width and 8 mm in seal width (see Figs. 1 and 2). Even after separating the trimming die from the heat generating composition molded body 2B, the heat generating composition molded body was free from a loss of shape, and collapsed pieces of the heat generating composition molded body were not generated in the surroundings of the heat generating composition molded body. Also, sealing could be completely carried out without causing incorporation of collapsed pieces of the heat generating composition molded body into the seal part, and seal failure did not occur. Incidentally, the air permeability of the covering material was 370 g/m$^2$/24 hr in terms of a moisture permeability by the Lyssy method. Next, the heat generating body was sealed and accommodated in an air-impermeable outer bag and then allowed to stand at room temperature for 24 hours. After 24 hours, the heat generating body was taken out from the outer bag and subjected to an exothermic test of heat generating body by the body. As a result, it was felt warm within 3 minutes, and thereafter, the warmth was continued for 10 hours or more.

(Example 10)

A stirring type batchwise oxidizing gas contact treatment device consisting of a mixer equipped with a rotary blade for stirring was used as an oxidizing gas contact treatment device, and air was used as an oxidizing gas. First of all, a reaction mixture consisting of 100 parts by weight of a reduced iron powder (particle size: not more than 300 $\mu$m), 5.2 parts by weight of active carbon (particle size: not more than 300 $\mu$m), 0.7 parts by weight of sodium sulfite, and 10 parts by weight of 11 % salt water and having a water mobility value of less than 0.01 was charged in the stirring type batchwise oxidizing gas contact treatment device. Next, in the state that the upper portion of the oxidizing gas contact treatment device was opened to air, the reaction mixture was subjected to self heat generation with stirring under circumstances at 20 °C and contact treated with an oxidizing gas at a maximum exothermic temperature of 68 °C until the exothermic temperature reached 35 °C, thereby obtaining a contact treated reaction mixture. With respect to the iron powder of the contact treated reaction mixture, an integrated intensity ratio was determined from an integrated intensity of peaks (at 58.28, 64.92 and 82.22 (2$\theta$/deg)) of a (110) plane of iron ($\alpha$Fe) and an integrated intensity of peaks (at 35.24, 41.59, 60.95, 72.70 and 76.51 (2$\theta$/deg)) of a (220) plane of FeO (wustite) by using an X-ray diffraction device, from which was then determined an amount of wustite. The amount of wustite of the reaction mixture was 10 % by weight. Next, 11 % salt water was mixed in the foregoing contact treated reaction mixture to obtain a heat generating composition having a water mobility value of 10.

(Comparative Example 6)

A heat generating composition having a water mobility value of 10 was prepared in the same manner as in Example 1, except that the contact treatment with an oxidizing gas was not carried out. Furthermore, a heat generating body was prepared in the same manner as in Example 1. The heat generating body was sealed and accommodated in an air-impermeable outer bag and allowed to stand at room temperature for 24 hours. After 24 hours, the heat generating body was taken out from the outer bag and subjected to an exothermic test of heat generating body. As a result, the temperature reached 24 °C within 3 minutes, and the exothermic rising properties were deteriorated.

(Example 11)

Each of the heat generating compositions as obtained in Example 10 and Comparative Example 6 was subjected to an exothermic test of heat generating composition. Comparative Example 6 was deteriorated in exothermic rising properties. The heat generating composition of Example 10 was excellent in exothermic rising properties.

(Example 12)

A stirring type batchwise oxidizing gas contact treatment device consisting of a mixer equipped with a rotary blade for stirring was used as an oxidizing gas contact treatment device, and air was used as an oxidizing gas. First of all, a reaction mixture consisting of 100 parts by weight of a reduced iron powder (particle size: not more than 300 $\mu$m), 5.2 parts by weight of active carbon (particle size: not more than 300 $\mu$m), 2. 3 parts by weight of a wood meal (particle size: not more than 300 $\mu$m), 2.3 parts by weight of a water absorptive polymer (particle size: not more than 300 $\mu$m), 0.2 parts by weight of calcium hydroxide, 0.7 parts by weight of sodium sulfite, and 10 parts by weight of 11 % salt water and having a water mobility value of less than 0.01 was charged in the stirring type batchwise oxidizing gas contact treatment device vessel. Next, in the state that the upper portion of the oxidizing gas contact treatment device vessel was opened to air, the reaction mixture was subjected to self heat generation with stirring under circumstances at 20 °C and contact treated with an oxidizing gas at a maximum exothermic temperature of 68 °C until the exothermic temperature reached 35 °C, thereby obtaining a contact treated reaction mixture. With respect to the iron powder of the contact treated reaction mixture, an integrated intensity ratio was determined from an integrated intensity of peaks of a (110) plane of iron ($\alpha$Fe) and an integrated intensity of peaks of a (220) plane of FeO (wustite) by using an X-ray diffraction device, from which was then determined an amount of wustite. The amount of wustite of the reaction mixture was 10 % by weight. Next, 11 % salt water was mixed in the foregoing contact treated reaction mixture to obtain a heat generating composition having a water mobility value of 8.

This heat generating composition was subjected to an exothermic test of heat generating composition. As a result, the temperature reached about 50 °C (an average value of five samples) after 3 minutes.

Furthermore, the heat generating composition was tested for moldability. As a result, even after separating a trimming die from a heat generating composition molded body, the heat generating composition molded body was free from a loss of shape, and collapsed pieces of the heat generating composition molded body were not generated in the surroundings of the heat generating composition molded body.

Next, a heat generating composition molded body was laminated on a polyethylene film of an air-impermeable substrate in which an adhesive layer provided with a separator was provided on the polyethylene film by force through molding using a trimming die having a rectangular cavity of 2 mm in thickness, 110 mm in length and 80 mm in width. In addition, an air-permeable covering material made of a laminate of a nylon-made non-woven fabric and a porous film was superimposed thereon such that the surface of the polyethylene film and the surface of the porous film were brought into contact with each other. The surroundings were heat sealed in a seal width of 8 mm and then cut to prepare a rectangular flat heat generating body of 130 mm in length, 100 mm in width and 8 mm in seal width. Even after separating the trimming die from the heat generating composition molded body, the laminate was free from a loss of shape, and collapsed pieces of the heat generating composition molded body were not generated in the surroundings of the heat generating composition molded body. Also, sealing could be completely carried out without causing incorporation of collapsed pieces of the heat generating composition molded body into the seal part, and seal failure did not occur.

Incidentally, the air permeability of the covering material was 370 $g/m^2/24$ hr in terms of a moisture permeability by the Lyssy method.

Next, the heat generating body was sealed and accommodated in an air-impermeable outer bag and then allowed to stand at room temperature for 24 hours.

After 24 hours, the heat generating body was taken out from the outer bag and subjected to an exothermic test of heat generating body by the body. As a result, it was felt warm after 3 minutes, and thereafter, the warmth was continued for 10 hours or more.

(Comparative Example 7)

A heat generating composition was obtained in the same manner as in Example 3, except that the contact treatment with an oxidizing gas was not carried out, from which was then obtained a heat generating body. The heat generating body was subjected to an exothermic test by a human body in the same manner as in Example 3. As a result, it took 6 minutes until it was felt warm.

With respect to Example 13 and Comparative Example 7, the exothermic test of heat generating body was carried out. As a result, in the case of Example 2, the temperature was 50 °C after 30 minutes and 58 °C after 3 hours, respectively. However, in the case of Comparative Example 7, the temperature was 45 °C after 30 minutes and 55 °C after 3 hours, respectively. The heat generating body using the heat generating composition of the invention was excellent in exothermic rising properties.

(Example 14)

100 parts by weight of an iron powder having a wustite content of 11 % by weight (particle size: not more than 300 $\mu$m), 5.3 parts by weight of active carbon (particle size: not more than 300 $\mu$m), 5 parts by weight of a wood meal (particle size: not more than 300 $\mu$m), 1.2 parts by weight of a water absorptive polymer (particle size: not more than 300 $\mu$m), 0.2 parts by weight of calcium hydroxide, 0.7 parts by weight of sodium sulfite, and 11 % salt water were mixed to obtain a heat generating composition having a water mobility value of 5.

Furthermore, the heat generating composition was tested for moldability. As a result, even after separating a trimming die from a heat generating composition molded body, the heat generating composition molded body was free from a loss of shape, and collapsed pieces of the heat generating composition molded body were not generated in the surroundings of the heat generating composition molded body.

Next, a heat generating composition as molded by means of force-through molding by a trimming die having a rectangular cavity of 2 mm in thickness, 110 mm in length and 80 mm in width was laminated on a polyethylene film of an air-impermeable substrate 3 in which an adhesive layer provided with a separator was provided on the polyethylene film, thereby obtaining a heat generating composition molded body. In addition, an air-permeable covering material made of a laminate of a nylon-made non-woven fabric and a porous film was superimposed thereon such that the surface of the polyethylene film and the surface of the porous film were brought into contact with each other. The surroundings were heat sealed in a seal width of 8 mm and then cut to prepare a rectangular flat heat generating body of 130 mm in length, 100 mm in width and 8 mm in seal width. Even after separating the trimming die from the heat generating composition molded body 2B, the laminate was free from a loss of shape, and collapsed pieces of the heat generating composition molded body were not generated in the surroundings of the heat generating composition molded body. Also, sealing could be completely carried out without causing incorporation of collapsed pieces of the heat generating composition molded body into the seal part, and seal failure did not occur. Incidentally, the air permeability of the covering material was 380 $g/m^2/24$ hr in terms of a moisture permeability by the Lyssy method.

Next, the heat generating body was sealed and accommodated in an air-impermeable outer bag and then allowed to stand at room temperature for 24 hours.

After 24 hours, the heat generating body was taken out from the outer bag and subjected to an exothermic test of heat generating body by the body. As a result, it was felt warm within 3 minutes, and thereafter, the warmth was continued for 10 hours or more.

(Example 14)

A heat generating composition having a water mobility value of 20 was obtained in the same manner as in Example 3, except for changing only the adjustment of the water content. Furthermore, the moldability of the heat generating composition was measured according to the foregoing measurement method for moldability. As a result, collapsed pieces of a heat generating composition molded body were not observed, and therefore, this heat generating composition was a moldable heat generating composition. Next, an air-impermeable packaging material having a 40 $\mu$m-thick air-impermeable polyethylene film stuck on one surface of a liner paper (thickness: 300 $\mu$m) was used as a substrate, and the foregoing heat generating composition was molded and laminated on the liner paper of the substrate by force-through molding using a trimming die having a rectangular cavity of 2 mm in thickness, 110 mm in length and 80 mm in width, thereby obtaining a heat generating composition molded body. In addition, a covering material in which a network-like adhesive layer provided with a styrene-isobutene-styrene block copolymer based sticky polymer in a network form by the melt blow method was provided in the side of a polyethylene-made porous film was superimposed thereon such that the network-like adhesive layer surface of the covering material and the heat generating composition molded body were brought into contact with each other. The surroundings of the heat generating composition molded body were sealed by compression seal, and the circumference was cut, thereby producing a rectangular flat heat generating body of 130 mm in length, 100 mm in width and 8 mm in seal width. The heat generating body was sealed in an outer bag which is an air-impermeable accommodating bag and then allowed to stand for 24 hours. Here, a laminate of a craft paper having a thickness of 30 $\mu$m, a polyethylene-made porous film having a thickness of 50 $\mu$m, and a nylon non-woven fabric having a thickness of 150 $\mu$m in this order was used as the covering material. The network-like adhesive layer was provided on the craft paper. Incidentally, the air permeability of the covering material was 650 g/m$^2$/24 hr in terms of a moisture permeability by the Lyssy method.

After 24 hours, the heat generating body was taken out from the outer bag and subjected to an exothermic test of heat generating body by the body. As a result, it was felt warm within 3 minutes, and thereafter, the warmth was continued for 10 hours or more.

As seen in Examples 1 to 14, the heat generating compositions and the heat generating bodies of the invention had satisfactory exothermic rising properties, were free from a lowering in exothermic characteristics due to compression, and were excellent in exothermic characteristics.

(Example 15)

Fig. 9 shows an embodiment of the force-through molding method using a leveling plate 15.

A substrate 3 in a roll film form having a width of 130 mm is adapted to a molding mold 12 having a thickness of 1 mm and having a desired shape punched out in the center thereof and horizontally sent at a prescribed speed between a die 11 as disposed in the upper surface and a magnet 13 as disposed in the lower surface. The heat generating composition 2 of Example 5 is sent into a mold hole 12a from the upper surface of the mold 12 through a hole 11a of the die 11. The heat generating composition is leveled in the same level as in the mold 12 by a leveling plate 15 as placed forward in the advancing direction and accommodated in the mold hole 12a, whereby a shape having a thickness of 1.5 mm is molded on the substrate 3. Thereafter, the mold 12 is removed to obtain a heat generating composition molded body as laminated on the substrate 3.

While not illustrated, a styrene-isoprene-styrene block copolymer (SIS) based sticky polymer is then provided in a network-like form on the surface of the heat generating composition molded body by the melt blow method, a covering material is covered thereon, and the periphery of the heat generating composition molded body is sealed by heat seal, followed by cutting into a desired shape. There is thus obtained a heat generating body having a desired shape. In addition, the cut heat generating body is subsequently sent into a packaging step and sealed in an air-tight outer bag. Furthermore, the same molding is possible even by changing the leveling plate 15 by a pushing leveling plate.

Fig. 10 shows a leveling plate 15, and Fig. 11 shows a pushing leveling plate 15A. Incidentally, so far as a pushing leveling function is kept, the tip of the pushing leveling plate may be rounded by trimming, namely, it may be deformed in any form by means of rounding, etc.

**Claims**

1. A heat generating composition, **characterized in that** the heat generating composition contains, as essential components, an iron powder, a carbon component, a reaction accelerator, a hydrogen formation inhibitor and water; that the iron powder contains from 20 to 100 % by weight of an active iron powder; and that the active iron powder is at least any one of:

    1) an active iron powder comprising particles, a surface of each of which is at least partially covered with an iron oxide film and a thickness of the iron oxide film is 3 nm or more, and which has a region of an oxygen-free iron component in at least one region selected from a central region of the particle and a region beneath the iron oxide film, and
    2) an active iron powder in which an amount of wustite is from 2 to 50 % by weight in terms of an X-ray peak intensity ratio to iron.

2. The heat generating composition according to claim 1, **characterized in that** the amount of wustite of the active iron powder is from 5.01 to 50 % by weight in terms of an X-ray peak intensity ratio to iron.

3. The heat generating composition according to claim 1, **characterized in that** the heat generating composition contains at least one member selected from additional components consisting of a water retaining agent, a water absorptive polymer, a pH adjusting agent, an aggregate, a fibrous material, a functional substance, a surfactant, an organosilicon compound, a pyroelectric substance, a moisturizer, a fertilizer component, a hydrophobic polymer compound, a heat generating aid, a metal other than iron, a metal oxide other than iron oxide, an acidic substance, and a mixture thereof.

4. The heat generating composition according to claim 1, **characterized in that** the heat generating composition has a water mobility value showing an amount of surplus water of less than 0.01.

5. The heat generating composition according to claim 1, **characterized in that** the heat generating composition contains surplus water having a water mobility value of from 0.01 to 20 and has moldability by the surplus water; and that the surplus water in the heat generating composition does not reveal a function as a barrier layer, whereby an exothermic reaction with air is not inhibited.

6. The heat generating composition according to claim 1, **characterized in that** the heat generating composition contains surplus water having a water mobility value of exceeding 20 and not more than 50 and has moldability by the surplus water; and that the surplus water in the heat generating composition reveals a function as a barrier layer, whereby an exothermic reaction with air is inhibited.

7. A heat generating body, **characterized in that** the heat generating composition according to claim 1 is accommodated in an air-permeable accommodating bag to form an exothermic part.

8. The heat generating body according to claim 7, **characterized in that** the accommodated heat generating composition is a heat generating composition molded body.

9. The heat generating body according to claim 7, **characterized in that** the accommodated heat generating composition forms sectional exothermic parts sectioned into plural sections by a seal part within the air-permeable accommodating bag; and that an exothermic part is formed from gathering of the sectional exothermic parts.

10. The heat generating body according to claim 9, **characterized in that** the sectional exothermic parts have a longest width of from 1 to 55 mm and a maximum height of from 0.1 to 10 mm; that a space of the sectional exothermic parts is from 0.5 to 30 mm; that the surroundings of the heat generating composition molded body are heat sealed to form sectional exothermic parts; that at least a part of the accommodating bag has air permeability; and that the periphery which becomes the heat generating body is sealed.

11. The heat generating body according to claim 9, **characterized in that** the heat generating composition molded body has a curved shape; and that the sectional exothermic parts have a curved shape and have a shortest radius of curvature of from 0.5 to 27.5 mm and a height of from 0.1 to 15 mm.

12. The heat generating body according to claim 7, **characterized in that** a fixing measure is provided on the exposed surface of the heat generating body and optionally provided with a separator.

13. A process for producing a heat generating body, **characterized by** accommodating a heat generating composition which contains, as essential components, an iron powder containing from 20 to 100 % by weight of at least one of:

1) an active iron powder comprising particles, a surface of each of which is at least partially covered with an iron oxide film and a thickness of the iron oxide film is 3 nm or more, and which has a region of an oxygen-free iron component in at least one region selected from a central region of the particles and a region beneath the iron oxide film, and

2) an active iron powder in which an amount of wustite is from 2 to 50 % by weight in terms of an X-ray peak intensity ratio to iron,

a carbon component, a reaction accelerator, a hydrogen formation inhibitor and water in an air-permeable accommodating bag.

14. The process for producing a heat generating body according to claim 13, **characterized in that** the heat generating composition contains at least one member selected from additional components consisting of a water retaining agent, a water absorptive polymer, a pH adjusting agent, a surfactant, an organosilicon compound, a hydrophobic polymer compound, a pyroelectric substance, an antioxidant, an aggregate, a fibrous material, a moisturizer, a functional substance, and a mixture thereof.

15. The process for producing a heat generating body according to claim 13, **characterized in that** the heat generating composition as accommodated in the air-permeable accommodating bag forms sectional exothermic parts sectioned into plural sections by a seal part within the air-permeable accommodating bag.

16. The process for producing a heat generating body according to claim 13, **characterized in that** the heat generating composition is a molded body, is provided in a packaging material made of a substrate and a covering material constituting the air-permeable accommodating bag and seals the surroundings of the heat generating composition molded body to produce a heat generating body.

*FIG.1*

*FIG.2*

*FIG.3*

# *FIG. 4*

# *FIG.5*

## FIG.6

## FIG.7

## FIG.8

## *FIG.9*

## *FIG.10*

## *FIG.11*

## FIG.12

## FIG.13

## FIG.14

# FIG.15

# FIG.16

<table>
<tr><td colspan="2" align="center">**INTERNATIONAL SEARCH REPORT**</td><td>International application No.<br>PCT/JP2005/013001</td></tr>
</table>

**A. CLASSIFICATION OF SUBJECT MATTER**
Int.Cl⁷ A61F7/08, C09K5/16

According to International Patent Classification (IPC) or to both national classification and IPC

**B. FIELDS SEARCHED**

Minimum documentation searched (classification system followed by classification symbols)
Int.Cl⁷ A61F7/08, C09K5/16

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2005 |
| Kokai Jitsuyo Shinan Koho | 1971-2005 | Toroku Jitsuyo Shinan Koho | 1994-2005 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP 2002-155273 A (Kaoru USUI),<br>28 May, 2002 (28.05.02),<br>Full text; Figs. 1 to 14<br>(Family: none) | 1-16 |
| A | JP 7-265347 A (Yukio KOMATSU),<br>17 October, 1995 (17.10.95),<br>Full text; Figs. 1 to 10<br>(Family: none) | 1-16 |
| A | JP 2003-102761 A (Kao Corp.),<br>08 April, 2003 (08.04.03),<br>Full text; Figs. 1 to 2<br>& US 2005/0000827 A1 & EP 1437111 A1<br>& WO 2003/028597 A1 & CN 1561187 A | 1-16 |

☒ Further documents are listed in the continuation of Box C. ☐ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| | |
|---|---|
| Date of the actual completion of the international search<br>24 August, 2005 (24.08.05) | Date of mailing of the international search report<br>13 September, 2005 (13.09.05) |
| Name and mailing address of the ISA/<br>Japanese Patent Office | Authorized officer |
| Facsimile No. | Telephone No. |

Form PCT/ISA/210 (second sheet) (January 2004)

**INTERNATIONAL SEARCH REPORT**

| | International application No. |
|---|---|
| | PCT/JP2005/013001 |

C (Continuation).   DOCUMENTS CONSIDERED TO BE RELEVANT

| Category* | Citation of document, with indication, where appropriate, of the relevant passages | Relevant to claim No. |
|---|---|---|
| A | JP 10-17907 A  (Dowa Iron Powder Co., Ltd.), 20 January, 1998 (20.01.98), Full text; Figs. 1 to 6 (Family: none) | 1-16 |

Form PCT/ISA/210 (continuation of second sheet) (January 2004)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 53060885 A
- JP 57010673 A
- JP 6343658 A
- JP 59189183 A
- JP 9075388 A
- JP 5081261 B
- JP 11508786 T
- JP 2002514104 T

- JP 3164605 B
- JP 2002200108 A
- JP 10265373 A
- JP 9087173 A
- JP 6145050 A
- JP 6199660 A
- JP 10279466 A
- JP 10182408 A